# EUROPEAN PATENT SPECIFICATION

(11) **EP 1 524 961 B1**
(45) Date of publication and mention of the grant of the patent: **21.08.2013**
(21) Application number: 03739096.0
(22) Date of filing: 12.06.2003
(51) Int. Cl.: A61K 8/06, A61K 8/891, A61K 8/893, A61K 8/894, A61K 8/898, A61K 8/899, A61Q 1/02, A61Q 1/04, A61Q 1/06, A61Q 1/12, A61Q 17/04, A61Q 19/00, A61Q 19/08

(54) **COSMETIC COMPOSITION FOR CARE AND/OR TREATMENT AND/OR MAKEUP OF THE EMULSION TYPE STRUCTURED WITH SILICONE POLYMERS**
KOSMETISCHE ZUSAMMENSETZUNG FÜR PFLEGE UND/ODER BEHANDLUNG UND/ODER MAKEUP VOM EMULSIONSTYP, STRUKTURIERT MIT SILIKONPOLYMEREN
COMPOSITION COSMETIQUE DE SOIN ET/OU DE TRAITEMENT ET/OU DE MAQUILLAGE DE TYPE EMULSION STRUCTUREE A L'AIDE DE POLYMERES DE SILICONE

(30) Priority: 12.06.2002 US 166648
(43) Date of publication of application: 27.04.2005
(73) Proprietor: L'Oréal, 75008 Paris (FR)
(72) Inventor: LU, Shaoxiang, Plainsboro, NJ 08536 (US)
(74) Representative: Le Coupanec, Pascale A.M.P.
(86) International application number: PCT/US2003/018503
(87) International publication number: WO 2003/105798

(56) References cited:
- EP-A- 1 266 647
- EP-A- 1 266 648
- EP-A- 1 266 653
- WO-A-02/17870
- WO-A-02/17871
- WO-A-03/013447
- US-A- 5 981 680
- US-A- 6 051 216
- US-A- 6 103 250

## Description

### Technical field

This invention relates to a cosmetic composition for care and/or treatment and/or makeup of the skin, including the scalp, and/or the lips of humans, of the emulsion type, containing a liquid fatty phase comprising at least one silicone oil, gelled, structured with a specific polymer. This composition exists notably in the form of a makeup product, in particular cast in a stick or in a dish, and most especially a concealer stick, body makeup, a foundation or lip product, the application of which results in a deposit without transfer, supple, flexible, natural, light, and with good staying power over time. Most especially, this composition is in the form of a solid water-in-oil (W/H) emulsion possessing excellent properties of flexibility, suppleness and non-transfer.

A cosmetic composition for care and/or treatment is a composition which comprises at least one active compound for treating wrinkles, for moisturizing the skin and the lips, for protecting the skin, the lips and the phaneric structures from ultraviolet rays, for treating acne and/or for acting as a self-tanning preparation.

The invention relates most especially to cosmetic and dermatological compositions such as makeup products, possessing properties of non-transfer, flexibility, suppleness, superior to those of known products.

### State of the prior art

In cosmetic or dermatological products, it is common to find a structured, that is, gelled and/or rigidified, liquid fatty phase; this is especially the case in solid compositions such as deodorants, lip balms and lipsticks, eye shadows, concealers, and foundations cast in a jar or in a dish. The products cast in a dish often are referred to as "compacts." This structuring is obtained with the aid of waxes or fillers. Unfortunately these waxes and-fillers have a tendency to make the composition matte, which is not always desirable, in particular for a lipstick or an eye shadow.

In the meaning of the invention, by "liquid fatty phase" is understood a fatty phase which is liquid at room temperature (25° C) and atmospheric pressure (760 mmHg), consisting of one or more non-aqueous compounds or fatty substances which are liquid at room temperature, also referred to as oils, generally mutually compatible and at least partially insoluble in an aqueous phase, said fatty phase containing at least one silicone oil.

In the meaning of the application, by structured liquid fatty phase it is understood that this structured phase does not flow under its own weight.

The structuring of the liquid fatty phase makes it possible in particular to limit its exudation from solid compositions, especially in hot and humid areas and, in addition, after deposition on the skin or the lips, to limit the migration of this phase into wrinkles and fine lines, which is particularly sought for a lipstick or an eye shadow.

This migration of the liquid fatty phase, particularly when it is considerable and this liquid fatty phase is charged with coloring agents, leads to an unsightly appearance around the lips or the eyes, in particular accentuating wrinkles and fine lines. This migration often is cited by women as a major drawback of conventional lipsticks or eye shadows. By "migration" is understood an extension of the composition deposited on the skin or the lips beyond its initial border.

Gloss basically is associated with the nature of the liquid fatty phase. Thus it is possible to reduce the level of waxes and-fillers in the composition in order to increase the gloss of a lipstick, but then the migration of the liquid fatty phase increases. In other words, the levels of waxes and of fillers required for the making of a stick of suitable hardness place a restriction on the gloss of the deposit.

Document EP-A-1 068 856 [1] describes solid cosmetic compositions, without wax, comprising a liquid fatty phase structured with a polymer, in which the fatty phase is mainly a non-silicone oil.

Document WO-A-01/97758 [2] describes cosmetic compositions based on polyamide resins comprising a gelling agent chosen from among the esters and amides of N-acylamino acids and mixtures thereof. The composition also comprises a polyamide resin solvent which may be chosen from among the saturated or unsaturated fatty alcohols, the esters of fatty and/or aromatic carboxylic acids, the ethoxylated and/or propoxylated alcohols and acids, the silicones, the mineral oils and the branched-chain hydrocarbons; preferably, the esters of fatty acids, the fatty alcohols, the mineral oils, the branched hydrocarbons and mixtures of the latter.

The use of fatty phases based on silicone oils has made it possible up to now to obtain cosmetic compositions having a long staying power when the oils are not very volatile or are nonvolatile, that is, a good staying power particularly of color in the course of time (non-changing, non-fading), and non-transfer compositions when the silicone oils are volatile, not becoming deposited on a support such as a glass, a cup, a fabric or a cigarette, placed in contact with the film of makeup.

At the present time, the use of silicone oils in cosmetics is limited by the lack of molecules capable of structuring and in particular gelling these mediums and thus resulting in compositions existing in solid form such as lipsticks or cast foundations, for example. The utilization of cosmetic compositions the fatty phase of which is mainly silicone in most cases results in problems of compatibility with the ingredients traditionally used in the cosmetics industry.

In documents US-A-5,874,069 [3], US-A-5,919,441 [4], US-A-6,051,216 [5], WO-A-02/17870 [6], and WO-A-02/17871 [7], WO-A-99/06473 [12], US-A-6,353,076 [13], cosmetic compositions such as deodorant sticks or gels were achieved comprising a silicone oily phase structured with a wax based on polysiloxane and polyamide, or with a polymer containing siloxane groups and groups capable of hydrogen interactions.

In WO-A-02/17870 [6], adding another gelling agent to the composition is contemplated, but the quantities added are to be small, for example less than 0.5% in the case of hydroxystearic acid, to preserve the clearness of the product.

In WO-A-02/17871 [7], using a second gelling agent with the silicone polymer in a quantity representing 0.5 to 2% by weight of the composition, and a solvent system comprising a non-silicone organic compound, a volatile silicone and possibly another silicone also is contemplated.

Document EP-A-1 177 784 [8] illustrates a deodorant composition comprising a liquid phase containing, for example, a volatile silicone and possibly a nonvolatile silicone and/or a non-silicone hydrophobic organic liquid, structured with an organic compound with amido groups, possibly with one or more polymer or non-polymer secondary structuring agents, in smaller proportions. Among the secondary structuring agents, this document mentions the polymers having siloxane groups and groups with hydrogen interactions without giving examples or results on a composition using these polymers.

It should be noted that documents [6], [7] and [8] relate to deodorants for which problems of exudation and migration of the liquid fatty phase charged with a coloring agent into the wrinkles and fine lines, as well as staying power, non-transfer, suppleness and flexibility of the composition do not arise as in the case of the makeup cosmetic products described hereinabove. In addition, gloss is not sought for deodorants.

Moreover, the sticks obtained by structuring the liquid fatty phase solely with one or more gelling silicone polymers do not afford a sufficient mechanical resistance to shearing, particularly when the stick is applied on the lips and/or the skin, resulting in a breaking of the stick.

In addition, cast makeup or care compositions more often than not exist in anhydrous form entailing, in particular on the face and on the body, but also on the eyelashes, phenomena of discomfort, stiffness, heaviness, greasiness and sometimes a mask-like or smothering feeling, which may become totally unacceptable.

In order to lighten the makeup and reduce these phenomena of heaviness and greasiness, cosmeticians have turned to foundation and lipstick compositions in the form of emulsions, which are of concern most particularly within the framework of this invention.

Thus, the company Shiseido, in its patent application EP-A-0 374 332, provided for lipstick and foundation compositions producing a fresh effect, in the form of a solid emulsion of the water-in-oil type containing a volatile silicone oil, a solid hydrocarbon wax, an organopolysiloxane modified with a polyoxyalkylene group, acting as an emulsifier of the aqueous phase in the fatty phase, and powder fillers. In document US-A-5,688,831, the company Procter & Gamble likewise has described moisturizing makeup compositions comprising one or more volatile silicones combined with one or more humectants, pigments and an organic amphiphilic compound capable of forming, on the skin or in the composition, smectic lyotropic liquid crystals containing said humectants.

Because of the presence of a high level of wax, these compositions still have the drawback of being heavy, of being difficult to smooth on, and more often than not, of imparting an unpleasant feel to the composition. In addition, the presence of these waxes generally causes the makeup to become matte, which is not always desirable for a lipstick or an eye shadow.

Finally, these compositions form stiff films or deposits, not supple at all, and have non-transfer properties which are still unsatisfactory.

A need remains, therefore, for a cosmetic composition, in particular in the form of an emulsion, not having the drawbacks of the compositions, and especially of the emulsions, hereinabove, and possessing excellent cosmetic properties, in particular properties of freshness, lightness, good staying power over time, not drying out the skin or the lips on which they are applied, at the time of application as well as in the course of time. In addition, this composition should be stable over time and easy to manufacture. The makeup obtained should be natural, comfortable, with no mask-like or smothering feeling, and homogeneous.

Above all, there still is a need for a cosmetic composition, especially an emulsion providing a film or deposit which is flexible, not stiff, and at the same time possessing excellent non-transfer properties, without its being necessary to incorporate substantial quantities of waxes into this composition.

In other words, there is a need for a cosmetic composition, in particular of the emulsion type, having a great stability over time, which transfers very little or not at all, that is, which is "without transfer" ("transfer-free") or resistant to transfer, and which also possesses excellent cosmetic properties, such as suppleness, non-rigidity, absence of brittleness, and a comfortable feeling to the film formed by said emulsion-type composition.

The transfer resistance of a cosmetic composition can be assessed by a test for transfer resistance well established in this field of the art, such as the placement of the made-up area against a piece of paper, 1 minute, 5 and 10 minutes after application of the makeup on the skin or the lips (so-called "kiss" test).

### Statement of the invention

The purpose of the invention is precisely to provide a composition, in particular a solid emulsion for care and/or makeup and/or treatment of the skin and/or the lips and/or the phaneric structures, making it possible to meet the needs, to satisfy the demands, to remedy the drawbacks, and to solve the problems mentioned previously in the description of the compositions of the prior art.

In a surprising manner, the applicant has found that the use of special polymers in the emulsions, in particular of the water-in-oil type, made it possible to structure, in the absence or presence of small quantities of wax, the silicone-oil-based liquid fatty phases of these emulsions, which are in the form of a cast or compact makeup or care product, and that the application of these emulsions resulted in a film having outstanding cosmetic properties.

In particular, the film is not stiff, brittle; it is supple, flexible, comfortable and gives a feeling of freshness, the non-transfer properties are excellent and, in any case, far superior to those of the compositions of the prior art. In addition, the composition, that is, the emulsion, is stable over time and does not exude at room temperature.

The emulsion-type composition according to the invention makes it possible to form a film possessing a set of excellent properties, and especially a combination of properties of flexibility, suppleness, freshness, on the one hand, and non-transfer on the other hand, which never has been obtained with the emulsion-type compositions of the prior art.

By "emulsion" is understood a composition containing an aqueous phase and a liquid fatty phase, one of the phases of which is dispersed in the other phase with or without emulsifier, the whole being homogeneous to the naked eye. By "solid emulsion" is understood an emulsion which does not flow under its own weight at room temperature and atmospheric pressure.

By stable is understood a composition which does not exude at room temperature (25° C) for at least 2 months, or even up to 9 months.

The use of these special polymers in emulsions, in particular solid or fluid emulsions, the fatty phase of which contains a silicone oil, makes it possible to obtain gels, in particular solid gels, having a good mechanical resistance and a correct rheology for allowing a deposit in sufficient quantity which is not sticky to the touch, affords a very good staying power, does not transfer (in particular when volatile silicone oils are used), does not migrate into wrinkles and fine lines, and is not stiff, brittle, that is, it may be described as flexible and supple.

The special polymers utilized according to the invention never have been used to structure, gel the fatty phase in the specific context, characteristic of the fatty phase of a particular cosmetic composition in the form of an emulsion.

Nothing led one to suppose that, in an unexpected manner, these special polymers were going to make it possible to obtain in such fatty phase the effects and advantages cited hereinabove, in particular as relates to non-transfer and flexibility.

That is all the more unexpected since this phase is a fatty phase of a specific composition comprising at least one silicone oil.

These surprising and unexpected effects, in particular in connection with the properties of non-transfer, flexibility, suppleness, are obtained basically by virtue of a special polymer of the invention, with small quantities of waxes, or even in the absence of the latter.

But there furthermore occurs a sort of synergy between the special polymer and the.silicone oil which brings about a simultaneous improvement of the properties of non-transfer, suppleness and flexibility.

The invention applies not only to products for making up the lips, such as lipsticks, lip glosses and lip pencils and for making up the skin, of the face as well as the human body, such as foundations possibly cast in a stick, jar or dish, concealer products, eye shadows or blushers and products for temporary tattooing, but also to products for the care and/or treatment of the skin, including the scalp, and the lips, especially such as the products in stick form for solar protection of the skin of the face and lips, the personal hygiene products especially such as deodorants in stick form, thickened shampoos and conditioners and products for making up the eyes such as eyeliners, cleansing products, especially in stick form, pencils and mascaras most especially cast in cake form, as well as products for making up or for care of the keratinous fibers such as the hair, the eyelashes and the eyebrows.

More precisely, the purpose of the invention is a cosmetic emulsion, in particular a solid emulsion, for care and/or makeup, characterized in that (i) it does not flow under its own weight at room temperature and atmospheric pressure, (ii) it comprises an aqueous phase and a liquid fatty phase dispersed one within the other, the said liquid fatty phase comprising at least one volatile silicone oil representing from 20 to 50 % by weight of the total weight of the emulsion and being chosen from within the group consisting of the following compounds: octyltrimethicone, hexyltrimethicone, Decamethyl cyclopentasiloxane D5, octamethyl cyclotetrasiloxane D4, dodecamethyl cyclohexasiloxane D6, heptamethyl octyltrisiloxane, decamethyl tetrasiloxane, dodecamethyl pentasiloxane, 1.5 cSt polydimethylsiloxane, 2 cSt polydimethylsiloxane, 3 cSt polymethylsiloxane, 5 cSt polydimethylsiloxane, wherein cSt is a viscosity unit equivalent to 10⁻⁶m²·s⁻¹, and mixtures thereof, and (iii) said liquid fatty phase is structured with at least one gelling polymer (homopolymer or copolymer) with a weight-average molecular mass ranging from 500 to 500,000, and containing at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among the ester, amide, sulfonamide, carbamate, thiocarbamate, urea, thiourea, oxamido, guanidino, biguanidino groups and combinations thereof, on condition that at least one of the groups is other than an ester group,
- the polymer being solid at room temperature and soluble in the liquid fatty phase at a temperature of 25 to 250°C;
- the aqueous phase, the liquid fatty phase and the gelling polymer forming a physiologically acceptable medium and,
characterized in that it comprises a coloring agent.

The composition of the invention can be in the form of a paste, a solid, more or less hard, deformable or otherwise, a more or less viscous cream.

It can be a simple emulsion, in particular oil-in-water (O/W) or water-in-oil (W/O); it also can consist of a multiple emulsion, for example a triple emulsion, oil-in-water-in-oil or water-in-oil-in-water or of a solid, rigid or soft gel with an oily continuous phase in which a phase not miscible with the oily phase, such as an aqueous phase, is dispersed.

The simple or multiple emulsion can comprise an aqueous or oily continuous phase possibly containing dispersed lipid vesicles. In particular, it is in the form of a solid emulsion cast in a stick or a dish. More particularly, it is in the form of a rigid emulsion, the liquid fatty phase forming the continuous phase.

The structuring of the liquid fatty phase can be modified depending on the nature of the polymer used, and can be such that a rigid structure in the form of a wand or a stick, with good mechanical resistance, is obtained.

These rigid compositions such as wands, when they are colored, make it possible, after application, to obtain a supple, non-heavy deposit having excellent non-transfer properties, more or less glossy, not migrating and with good staying power, especially of the color, over time, and/or not transferring. The composition can contain one or more structuring polymers.

The emulsion according to the invention advantageously is a foundation or mascara composition, in which the excellent and improved properties relating to non-transfer, flexibility and suppleness are particularly underscored.

### Liquid fatty phase

According to the invention, the liquid fatty phase(s) includes/include at least one volatile silicone oil

The liquid fatty phase can comprise one volatile silicone oil and at least one volatile non-silicone oil.

According to the invention, the volatile silicone oil can be chosen from among the linear or cyclic silicone oils having a flash point equal to or in excess of 40°C and/or a viscosity below 8 cSt, such as the linear or cyclic polydimethyl siloxanes (PDMS) having from 3 to 7 silicon atoms.

The fatty phase advantageously can be composed solely of such volatile silicone oil or oils.

The flash point is the temperature at which a fuel ignites on contact with a flame.

The volatile oil advantageously has a flash point in excess of 60°C, preferably in excess of 80°C and better still in excess of 93°C.

The volatile oil also preferably has a flash point equal to or less than 135°C.

By "volatile oil" is understood any non-aqueous medium capable of evaporating on contact with the skin in less than one hour, at a room temperature and atmospheric pressure. In the meaning of the invention, a volatile silicone or non-silicone oil generally has a flash point preferably from 40 to 135°C or no flash point. The volatile cosmetic oil or oils, liquid at room temperature, have in particular a vapor pressure, measured at room temperature (25°C) and atmospheric pressure (760 mmHg) ranging from 10⁻³ to 300 mmHg (0.266 Pa to 40,000 Pa), preferably from 0.02 mmHg to 300 mmHg (2.66 PA to 40,000 Pa) and better ranging from 0.1 to 90 mmHg (13 Pa to 12,000 Pa). The nonvolatile oils then correspond to a vapor pressure of less than 0.02 mmHg (2.66 Pa).

The silicone oils of the invention have a viscosity advantageously chosen in the range from 5 to 800,000 cSt at 25°C, preferably from 10 to 500,000 cSt, and better from 10 to 5,000 cSt.

The volatile oil, when it is present, represents from 20 to 50% of the total weight of the emulsion.

According to the invention, the volatile silicone oil can be chosen from among the linear or cyclic silicone oils having a flash point of at least 40°C, such as the linear or cyclic polydimethylsiloxanes having from 2 to 7 silicon and possibly comprising an alkyl chain pendent or at the end of the chain having from 2 to 10 carbon atoms, which chain can be linear or branched.

As examples of such volatile oils, there may be cited the compounds given in Table 1 hereinbelow.

**Table 1**

| **Compound** | **Flash point** | **viscosity** |
|---|---|---|
| | **(°C)** | **(cSt)** |
| Octyltrimethicone | 93 | 1.2 |
| Hexyltrimethicone | 79 | 1.2 |
| Decamethyl cyclopentasiloxane (cyclopentasiloxane or D5) | 72 | 4.2 |
| Octamethylcyclo tetrasiloxane (cyclotetradimethyl siloxane or D4) | 55 | 2.5 |
| Dodecamethylcyclo hexasiloxane (D6) | 93 | 7 |
| Decamethyltetrasiloxane (L4) | 63 | 1.7 |
| KF 96 A from Shin Etsu | 94 | 6 |
| PDMS (polydimethylsiloxane) DC 200 (1.5 cSt) from Dow Corning | 56 | 1.5 |
| PDMS DC 200 (2 cSt) from Dow Corning | 87 | 2 |
| PDMS DC 200 (5 cSt) from Dow Corning | 134 | 5 cSt |
| PDMS DC 200 (3 cSt) from Dow Corning | 102 | 3 cSt |

In other words, the volatile silicone oil(s) may be chosen from within the group composed of the compounds of Table 1, heptamethyloctyltrisiloxane, dodecamethylpentasiloxane and mixtures thereof.

The composition of the invention may also comprise a volatile silicone chosen from within the group of fluorous silicone oils such as the silicones with alkyl and perfluoralkyl groups.

According to the invention, the liquid fatty phase can contain one or more volatile or nonvolatile non-silicone oils. The volatile non-silicone oils can be chosen from within the group of hydrocarbon oils and volatile esters and ethers such as the volatile hydrocarbons like isododecane and isohexadecane, the C₈-C₁₆ isoparaffins, the isohexyl or isodecyl neopentanoates, and mixtures thereof.

The volatile non-silicone oil also may be chosen from among the fluorous oils such as the perfluoropolyethers, the perfluoroalkanes such as perfluorodecaline, the perfluorodamantanes, the monoesters, diesters and triesters of perfluoroalkyl-phosphates and the fluorous ester oils.

As examples of volatile non-silicone oils which can be used in the invention, there may be cited the compounds of Table 2 which follows.

**Table 2**

| **Compound** | **Flash point** |
|---|---|
| | **(°C)** |
| Isododecane | 43 |
| Isohexadecane | 102 |
| Isodecyl neopentanoate | 118 |
| propylene glycol n-butylether | 60 |
| 3-ethyl ethoxypropionate | 58 |
| Propylene glycol methylether acetate* | 46 |
| Isopar L (C₁₁-C₁₃ isoparaffin) | 62 |
| Isopar H (C₁₁-C₁₂ isoparaffin) | 56 |

The liquid fatty phase advantageously contains at least 30% and better still at least 40% by weight of silicone oil(s) advantageously having a viscosity of less than 1,000 cSt and better less than 100 cSt, because the silicone polymers used in the invention are more soluble in the low-viscosity silicone oils. It also can contain other oils or a mixture of non-silicone oils.

The nonvolatile silicone oils can be polydimethylsiloxanes, polyalkylmethylsiloxanes, dimethicone copolyols, alkylmethicone copolyols, cetyldimethicone, silicones with alkylglyceryl ether groups, silicones with side amine groups and dilauroyltrimethylol propane siloxysilicate. The alkyl groups of these oils have in particular from 2 to 24 carbon atoms.

The nonvolatile silicone oils which can be used in the invention can be, in particular, nonvolatile linear polydimethylsiloxanes (PDMS) liquid at room temperature; polydimethylsiloxanes containing alkyl, alcoxy or phenyl groups, pendent and/or at the end of the silicone chain, groups each having from 2 to 24 carbon atoms; phenylated silicones such as the phenyl trimethicones, the phenyl dimethicones, the phenyl trimethylsiloxy diphenylsiloxanes, the diphenyl dimethicones, the diphenyl methyldiphenyl trisiloxanes, and the 2-phenylethyl trimethylsiloxysilicates, the fluorous silicones with pendent or end-chain group(s) having from 1 to 12 carbon atoms in which all or part of the hydrogen atoms are substituted with fluorine atoms, the dimethiconols and mixtures thereof.

The liquid fatty phase also can contain other non-silicone oils, for example polar oils such as:
- the hydrocarbon vegetable oils with a high triglyceride content composed of esters of fatty acids and glycerol, the fatty acids of which can have varying chain lengths, for example C₄-C₂₄, the latter being able to be linear or branched, saturated or unsaturated; these oils are in particular wheat germ oil, corn oil, sunflower oil, karite butter, castor oil, sweet almond oil, macadamia oil, apricot oil, soybean oil, rape oil, cotton oil, alfalfa oil, poppy oil, pumpkin oil, sesame oil, marrow oil, avocado oil, hazelnut oil, grapeseed or blackcurrant seed oil, evening primrose oil, millet oil, barley oil, quinoa oil, olive oil, rye oil, safflower oil, candlenut oil, passion flower oil musk rose oil; or else caprylic/capric acid triglycerides such as those sold by the company Stearines Dubois or those sold under the names Miglyol 810, 812 and 818 by the company Dynamit Nobel;
- the synthetic oils or esters of formula R₅COOR₆ in which R₅ represents the residue of a linear or branched higher fatty acid containing from 1 to 40 and better from 7 to 19 carbon atoms and R₆ represents a branched hydrocarbon chain containing from 1 to 40 and better from 3 to 20 carbon atoms, with R₅ + R₆ > 10, such as, for example, purcellin oil (cetostearyl octanoate), isononyl isononanoate, C₁₂ to C₁₅ alkyl benzoate, isopropyl myristate, 2-ethylhexyl palmitate, alkyl or polyalkyl octanoates, decanoates or ricineolates; hydroxylated esters such as isostearyl lactate, diisostearyl malate; and pentaerythritol esters;
- nonvolatile oils of the synthetic ester oil type can be chosen for example, from among glyceryl triisostearate or glycerol trioctanoate, diisostearate dimerate, diisopropyl dimer dilinoleate, glycerol lesquerolate and diisostearate dimerate;
- synthetic ethers having from 10 to 40 carbon atoms;
- C₈ to C₂₆ fatty alcohols such as oleic alcohol or octyldodecanol;
- fatty acids such as oleic acid, linoleic acid or linolenic acid; and
- mixtures thereof.

The liquid fatty phase also can contain apolar oils such as linear or branched hydrocarbons or fluorocarbons of synthetic or mineral origin, volatile or nonvolatile, such as volatile paraffin oils (such as isoparaffins, isododecane) or nonvolatile paraffin oils and derivatives thereof, vaseline, polydecanes, hydrogenated polyisobutene such as Parleam7 (sold by the company Nippon Oil Fats), squalane, and mixtures thereof.

Thus the invention can be implemented, for example, with the following different fatty phases:
1) a fatty phase consisting of a mixture of oils comprising at least one nonvolatile silicone oil and at least one volatile silicone oil;
2) a fatty phase consisting of a mixture of oils comprising at least one nonvolatile silicone oil, at least one volatile silicone oil and at least one volatile non-silicone oil;
3) a fatty phase consisting of a mixture of oils comprising at least one volatile silicone oil, one nonvolatile non-silicone oil and possibly at least one volatile non-silicone oil, and
4) a fatty phase consisting solely of volatile silicone oil(s).

In cases 1) and 2), the mixture also can comprise a nonvolatile non-silicone oil.

The liquid fatty phase generally represents from 5 to 98.4% of the total weight of the emulsion, and better from 20 to 75%.

The aqueous phase generally represents from 1 to 94.4% and preferably from 5 to 80% of the total weight of the emulsion, and better still from 20 to 60%.

### Solid particles

According to the invention, the composition generally furthermore comprises solid particles chosen from among fillers, pigments (including nacreous pigments), and mixtures thereof. Generally the average size of the solid particles is from 10 nm to 50 *µ*m, and better from 50 nm to 30 *µ*m, for example from 100 nm to 10 *µ*m.

The fillers used in cosmetic compositions generally have the purpose of absorbing sweat and sebum and/or imparting a matte appearance. According to the invention, they furthermore make it possible to structure the liquid fatty phase comprising a volatile silicone oil and to intensify the properties of staying power and/or non-transfer of the composition as well as the thermal stability.

By pigments is understood any solid particle insoluble in the composition serving to impart and/or to modify a color and/or an iridescent appearance.

These pigments can ensure both the function of absorption of sweat and sebum, and the function of coloring or of modification of the appearance of the composition, that is, of the makeup and/or care cosmetic product. In the invention, they also ensure the structuring of the liquid fatty phase.

These fillers or pigments can be of either hydrophobic or hydrophilic nature. When these fillers or pigments are hydrophilic particles, their dispersion in the composition is facilitated either by coating them in a film of hydrophobic compound or by adding a dispersant, and in particular a silicone amphiphilic with respect to the composition.

The solid particles, that is, the hydrophobic pigments or fillers, can be composed of hydrophobic polymer or copolymer powders. As examples of hydrophobic polymers and copolymers used as fillers, there may be cited:
1) fluorous polymers such as polytetrafluoroethylene powders and powders of tetrafluoroethylene and olefin copolymer, for example of ethylene or propylene; 2) silicone elastomers, for example polymethylsilsesquioxane powders (Tospearl^{®} from Toshiba); 3) polyolefins such as polyethylene; 4) alkyl polymethacrylates, for example methyl polymethacrylate; 5) polyamides (Nylon^{®}); 6) polystyrenes; 7) polyesters and derivatives thereof; 8) polyacrylics (Polytrap^{®} from Dow Corning) or methyl polymethacrylate (GANZPEARL GMX-0610); and 9) polyurethanes, for example powders of Hexamethylene Diisocyanate/trimethylol hexalactone.

There also can be used hydrophilic fillers treated on the surface so as to be hydrophobic, such as boron nitride, starch, precipitated calcium carbonate, silica, glass or a ceramic.

Instead of powders, there of course can be used fibers of a hydrophobic nature, in particular fibers of the polymers and copolymers cited previously.

The solid particles also can be composed of pigments and/or nacres making it possible to obtain a covering makeup, that is, not allowing the skin, lips or phaneric structures to show through. These particles furthermore make it possible to reduce the sticky feel of the compositions.

The pigments can be white or colored, organic and/or inorganic, coated or uncoated. The inorganic pigments can be chosen, for example, from among the zinc oxides, iron oxides, titanium oxides and mixtures thereof.

Thus among the inorganic pigments, there may be cited titanium or zinc dioxide, possibly surface-treated, zirconium or cerium oxides, as well as iron or chromium oxides, manganese violet, ultramarine blue, chromium hydrate and ferric blue. Among the organic pigments there may be cited carbon black, pigments of the D & C type, and the lakes based on cochineal carmine, on barium, strontium, calcium, aluminum. The pigments can represent from 0.1 to 50%, preferably from 0.5 to 40% and better from 2 to 30% of the total weight of the composition, if they are present.

The nacreous pigments (or nacres) can be chosen from among the white nacres such as mica coated with titanium or with bismuth oxychloride, colored nacreous pigments such as titanium mica with iron oxides, titanium mica with, in particular, ferric blue or chromium oxide, titanium mica with an organic pigment of the type mentioned above, as well as nacreous pigments based on bismuth oxychloride. They can represent from 0.1 to 20% of the total weight of the composition, and better from 0.1 to 15%, if they are present.

When the pigments or the fillers are hydrophilic, they are coated in a film of hydrophobic compound with a view to introducing them into the liquid fatty phase of the composition of the invention.

The coating can be a fluorous coating such as a perfluoroalkyl mono- or diester of phosphoric acid (acid or salt), a perfluoropolyether, a carboxylic or sulfonic perfluoroacid, or a salt of diethanolamine perfluoroalkyl phosphate.

The coating can be a coating based on fluorous silicone, for example a coating-grafting with a silane with a perfluoroalkyl group.

The coating also can be implemented by means of silicone derivatives, for example a coating-grafting with reactive silicones initially possessing hydrogenosilane groups, a coating grafting with a diorganosilane such as dimethylchlorosilane or with an alkylalcoxysilane, a coating-grafting with a silane with a glycydoxypropyl group, a coating with a polyglycerol silicone , or a coating with a silicone or silicone-g-polyacrylic grafted acrylic copolymer.

There also can be used a coating with N-acylamino acids, for example N-lauroyllysine, coatings with fatty acids or fatty acid salts of the stearic acid type, coatings with lecithins and coatings with ester oils.

Dispersion of the hydrophilic particles also may be facilitated by means of at least one amphiphilic silicone which serves as a surfactant between the hydrophilic particles and the hydrophobic silicone phase.

These amphiphilic silicones contain a silicone portion which is compatible with the highly silicone medium of the compositions of the invention, and a hydrophilic portion which can be, for example, the residue of a compound chosen from among the alcohols and the polyols, having from 1 to 12 hydroxyl groups, the polyoxyalkylenes containing at least two oxyalkylene moieties and having from 0 to 20 oxypropylene moieties and/or from 0 to 20 oxyethylene moieties. This hydrophilic portion therefore has an affinity for the hydrophilic particles and contributes to the dispersion thereof in the silicone medium.

The amphilic silicone can be an oil without gelling activity. Such oils can be composed of:
- dimethicone copolyols, possibly containing phenyl groups,
- alkylmethicone copolyols,
- polyglycerol silicones, that is, silicones with alkylglyceryl ether groups,
- silicones with perfluorous side groups and with glycerol side groups,
- silicones with polyoxyethylene/polyoxypropylene side groups and with perfluorous side groups,
- silicone block and hydrophilic block copolymers other than polyether, for example polyoxazoline or polyethyleneimine,
- grafted copolymers of the silicone grafted polysaccharide type,
- silicone block, poly(ethylene oxide/propylene oxide) block copolymers.

The amphiphilic silicone used in the invention also can be an at least partially crosslinked amphiphilic silicone resin.

As examples of such resins, there can be cited:
- crosslinked silicon resins with alkylpolyether groups, such as ethylene polyoxide (POE) and ethylene polyoxide/propylene polyoxide (POE/POP), described in US-A-5,412,004 [9], and
- silicone resins crosslinked in part with α,ω-dienes, possessing both hydrophilic POE/POP side chains and hydrophobic alkyl side chains such as those described in EP-A-1 048 686 [10]. The hydrophilic side chains are obtained by reaction with a POE/POP at a single vinyl end, and the alkyl side chains are formed by reaction with an α-olefin with a fatty chain (namely a C₈ to C₃₂ chain).

In the amphiphilic silicone resin, the silicone portion advantageously is made up of polydimethylsiloxane.

### Gelling silicone polymer

The polymer or polymers structuring or gelling the liquid fatty phase of the emulsion of the invention is/are solid at room temperature (25° C) and atmospheric pressure (760 mmHg) and soluble in the liquid fatty phase at a temperature of 25 to 250° C. "Soluble," in the meaning of the invention, signifies that the polymer and silicone oil mixture has at least a softening point.

In the meaning of the invention, there is understood by polymer a compound having at least 2 repeating moieties, preferably at least 3 repeating moieties, and better still 10 repeating moieties.

In the composition of the invention, the silicone polymer represents (in active substance) generally from 0.5 to 80%, preferably from 2 to 60%, and better still from 5 to 40% of the total weight of the composition.

Furthermore, the gelling polymer silicone oil(s) mass ratio is preferably from 0.1 to 50%.

The polymers used as gelling agents in the composition of the invention are polymers of the polyorganosiloxane type such as those described in the documents US-A-5,874,069 [3], US-A-5,919,441 [4], US-A-6,051,216 [5] and US-A-5,981,680 [11].

According to the invention, the polymers used as a gelling, structuring agent can belong to the following two families:
1) polyorganosiloxanes comprising at least two groups capable of establishing hydrogen interactions, these two groups being located in the polymer chain; and/or
2) polyorganosiloxanes comprising at least two groups capable of establishing hydrogen interactions, these two groups being located on grafts or branches.

The polymers to which the invention applies are solids that can be dissolved beforehand in a solvent with hydrogen interaction capable of breaking the hydrogen interactions of the polymers, such as the C₂ to C₈ lower alcohols and in particular ethanol, n-propanol, isopropanol, before being placed in the presence of the silicone oils according to the invention. It also is possible to use these hydrogen interaction "breaking" solvents as cosolvent. These solvents then can be kept in the composition or else be removed by selection evaporation, well known to the person skilled in the art.

The polymers comprising two groups capable of establishing hydrogen interactions in the polymer chain can be polymers comprising at least one moiety corresponding to the formula: 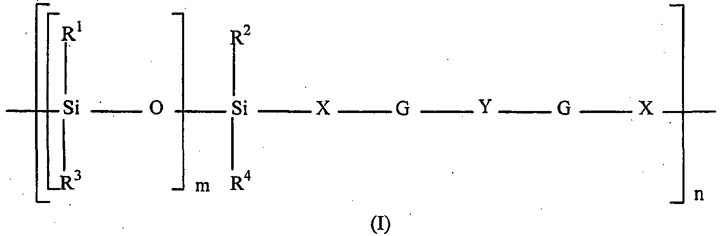 in which:
1) R¹, R², R³ and R⁴, identical or different, represent a group chosen from among:
   - linear, branched or cyclic, saturated or unsaturated, C₁ to C₄₀ hydrocarbon groups, which can contain in their chain one or more oxygen, sulfur and/or nitrogen atoms, and which can be partly or totally substituted with fluorine atoms,
   - C₆ to C₁₀ aryl groups, possibly substituted with one or more C₁ to C₄ alkyl groups,
   - polyorganosiloxane chains containing or not containing one or more oxygen, sulfur and/or nitrogen atoms;
2) the groups X, identical or different, represent a linear or branched C₁ to C₃₀ alkylenediyl group, which can contain in its chain one or more oxygen and/or nitrogen atoms;
3) Y is a saturated or unsaturated, C₁ to C₅₀ linear or branched divalent alkylene, arylene, cycloalkylene, alkylarylene or arylalkylene group, which can comprise one or more oxygen, sulfur and/or nitrogen atoms, and/or bear as substituent one of the following atoms or groups of atoms: fluorine, hydroxy, C₃ to C₈ cycloalkyl, C₁ to C₄₀ alkyl, C₅ to C₁₀ aryl, phenyl possibly substituted with 1 to 3 C₁ to C₃ alkyl groups, C₁ to C₃ hydroxyalkyl and C₁ to C₆ aminoalkyl, or
4) Y represents a group corresponding to the formula: 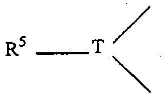 in which
   - T represents a linear or branched, saturated or unsaturated, C₃ to C₂₉ trivalent or tetravalent hydrocarbon chain, possibly substituted with a polyorganosiloxane chain, and which can contain one or more atoms chosen from among O, N and S, or T represents a trivalent atom chosen from among N, P and Al, and
   - R₅ represents a linear or branched C₁ to C₅₀ alkyl group, or a polyorganosiloxane chain, which can comprise one or more ester, amide, urethane, thiocarbamate, urea, thiourea and/or sulfonamide groups which can be linked or not linked to another chain of the polymer,
5) the groups G, identical or different, represent divalent groups chosen from among: 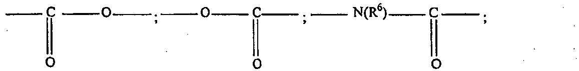 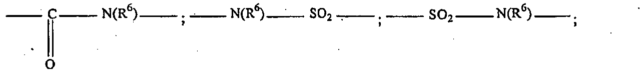 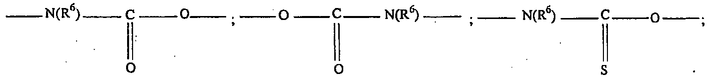 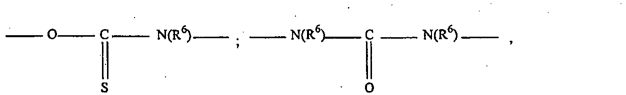 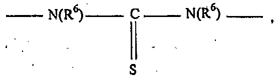 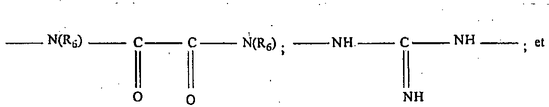 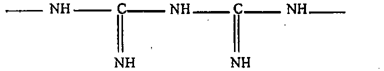 in which R₆ represents a hydrogen atom or a linear or branched C₁ to C₂₀ alkyl group, on condition that at least 50% of the groups R⁶ of the polymer represents a hydrogen atom and that at least two of the groups G of the polymer are a group other than: 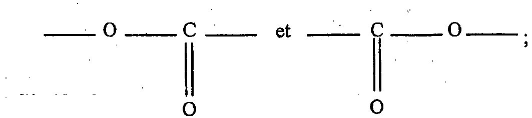
6) n is an integer ranging from 2 to 500, preferably from 2 to 200, and m is an integer ranging from 1 to 1000, preferably from 1 to 700, and better still from 6 to 200.

According to the invention, 80% of the groups R¹, R², R³ and R⁴ of the polymer preferably are chosen from among the methyl, ethyl, phenyl and 3,3,3-trifluoropropyl groups.

According to the invention, Y can represent various divalent groups, possibly furthermore comprising one or two free valences to establish bonds with other moieties of the polymer or copolymer. Preferably Y represents a group chosen from among:
a) linear C₁ to C₂₀, preferably C₁ to C₁₀, alkylene groups,
b) C₃₀ to C₅₆ branched alkylene groups which can comprise rings and unconjugated unsaturations,
c) C₅-C₆ cycloalkylene groups,
d) phenylene groups possibly substituted with one or more C₁ to C₄₀ alkyl groups,
e) C₁ to C₂₀ alkylene groups, comprising from 1 to 5 amide groups,
f) C₁ to C₂₀ alkylene groups comprising one or more substituents chosen from among the hydroxyl, C₃ to C₈ cycloalkane, C₁ to C₃ hydroxyalkyl and C₁ to C₆ alkylamine groups,
g) polyorganosiloxane chains of formula: 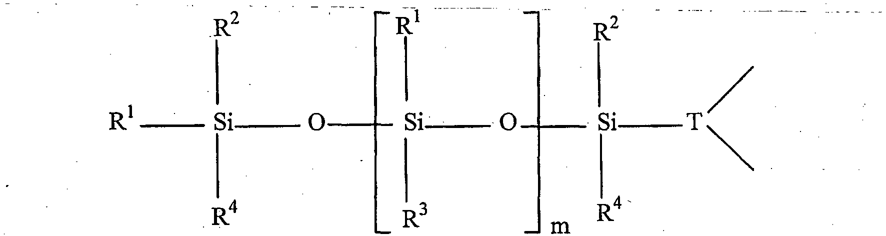 in which R¹, R² R³, R⁴, T and m are such as defined hereinabove, and
h) polyorganosiloxane chains of formula:

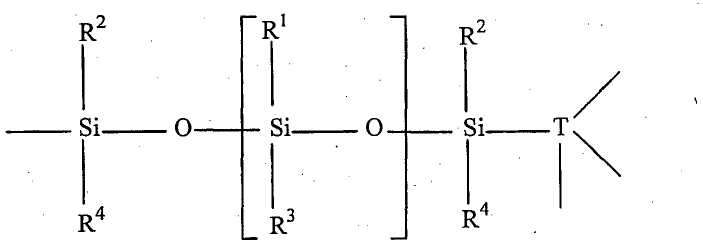

The polyorganosiloxanes of the second family can be polymers comprising at least one moiety corresponding to formula (II): 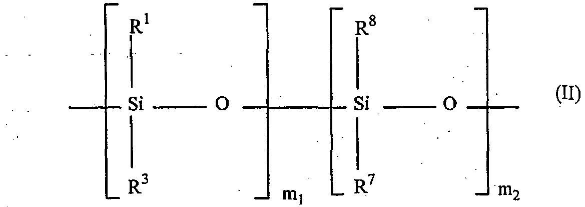 in which
- R¹ and R³, identical or different, are such as defined hereinabove for formula (I),
- R⁷ represents a group such as defined hereinabove for R¹ and R³, or represents the group of formula -X-G-R⁹ in which X and G are such as defined hereinabove for formula (I) and R⁹ represents a hydrogen atom or a linear, branched or cyclic, saturated or unsaturated, C₁ to C₅₀ hydrocarbon group, possibly comprising in its chain one or more atoms chosen from among O, S and N, possibly substituted with one or more fluorine atoms and/or one or more hydroxyl groups, or a phenyl group possibly substituted with one or more C₁ to C₄ alkyl groups.
- R⁸ represents the group of formula -X-G-R⁹ in which X, G and R⁹ are such as defined hereinabove,
- m₁ is an integer ranging from 1 to 998, and
- m₂ is an integer ranging from 2 to 500.

According to the invention, the polymer used as a gelling agent can be a homopolymer, that is, a polymer comprising several identical moieties, in particular moieties of formula (I) or of formula (II).

According to the invention, there also may be used a polymer consisting of a copolymer comprising several different moieties of formula (I), that is, a polymer in which at least one of the groups R¹, R², R³, R⁴, X, G, Y, m and n is different in one of the moieties. The copolymer also can be made up of several moieties of formula (II), in which at least one of the groups R¹, R³, R⁷, R⁸, m₁ and m₂ is different in at least one of the moieties.

There also may be used a copolymer comprising at least one moiety of formula (I) and at least one moiety of formula (II), the moieties of formula (I) and the moieties of formula (II) being able to be identical or different from one another.

According to a variant of the invention, there also may be used a copolymer furthermore containing at least one hydrocarbon moiety comprising two groups capable of establishing hydrogen interactions, chosen from among the ester, amide, sulfonamide, carbamate, thiocarbamate, urea, thiourea, oxamido, guanidino, biguanidind groups and combinations thereof. These copolymers may be block copolymers, sequenced copolymers or grafted copolymers.

According to a first embodiment of the invention, the groups capable of establishing hydrogen interactions are amide groups of formula -C(O)NH- and -HN-C(O)-.

In this case, the gelling agent can be a polymer comprising at least one moiety of formula (III) or (IV): 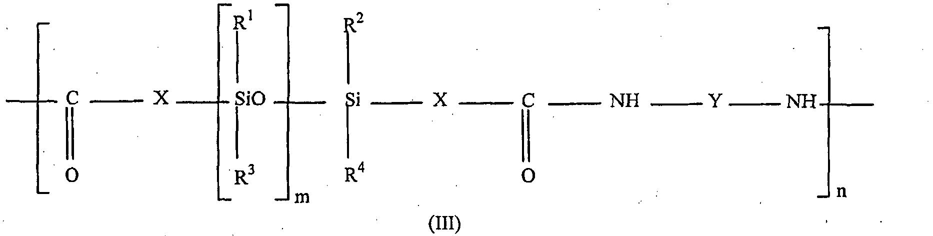 or 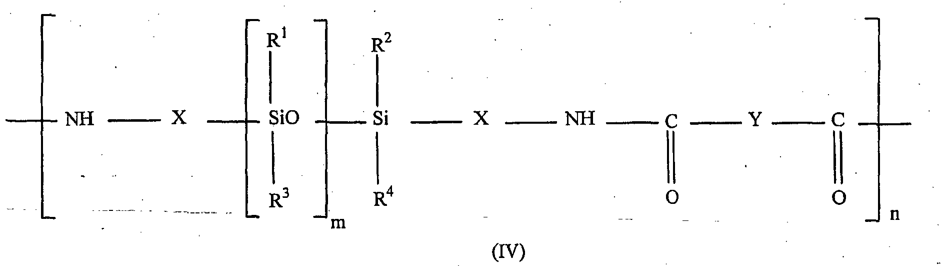 in which R¹, R², R³, R⁴, X, Y, m and n are such as defined hereinabove.

Such a moiety can be obtained:
- either by a condensation reaction between a silicone with α,ω-carboxylic acid ends and one or more diamines, according to the following reaction diagram: 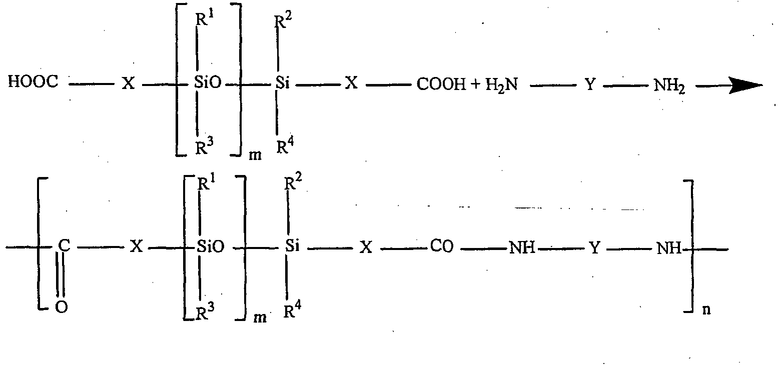
- or by reaction of two molecules of α-unsaturated carboxylic acid with a diamine according to the following reaction diagram:

   CH₂=CH-X¹-COOH+H₂N-Y-NH₂ → CH₂=CH-X¹-CO-NH-Y-NH-CO-X¹-CH=CH₂

   followed by the addition of a siloxane to the ethylene unsaturations, according to the following diagram: 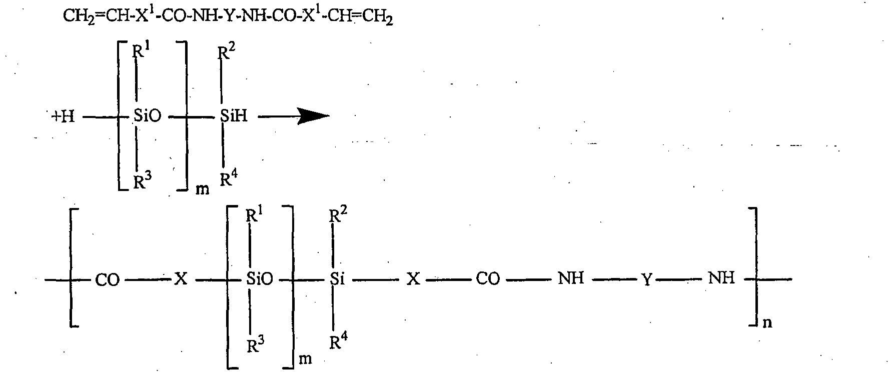 in which X¹-(CH₂)₂- corresponds to X defined hereinabove and Y, R¹, R², R³, R⁴ and m are such as defined hereinabove;
- or by reaction of a silicone with α,ω-NH₂ ends and a diacid of formula HOOC-Y-COOH according to the following reaction diagram: 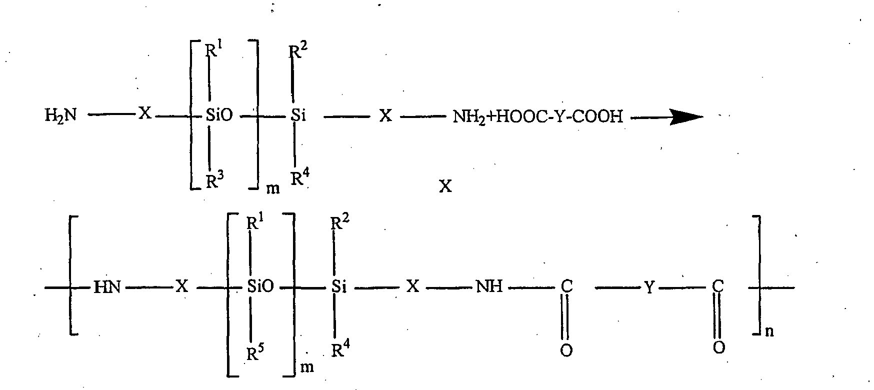 In these polyamides of formula (III) or (IV), m is preferably in the range from 1 to 700, preferably from 15 to 500 and better still from 15 to 45, and n in particular is in the range from 1 to 500, preferably from 1 to 100 and better still from 4 to 25,
- X is preferably a linear or branched alkylene chain having 1 to 30 carbon atoms, in particular 3 to 10 carbon atoms, and
- Y is preferably an alkylene chain that is linear or branched or that can comprise rings and/or unsaturations, having from 1 to 40 carbon atoms, in particular from 1 to 20 carbon atoms, and better still from 2 to 6 carbon atoms, in particular 6 carbon atoms.

In formulas (III) and (IV), the alkylene group representing X or Y possibly can contain at least one of the following elements in its alkylene portion:
1) 1 to 5 amide, urea or carbamate groups,
2) a C₅ or C₆ cycloalkyl group, and
3) a phenylene group possibly substituted with 1 to 3 identical or different C₁ to C₃ alkyl groups.

In formulas (III) and (IV), the alkylene groups also may be substituted with at least one element chosen from within the group consisting of:
- a hydroxy group,
- a C₃ to C₈ cycloalkyl group,
- one to three C₁ to C₄₀ alkyl groups,
- a phenyl group possibly substituted with one to three C₁ to C₃ alkyl groups,
- a C₁ to C₃ hydroxyalkyl group, and
- a C₁ to C₆ aminoalkyl group.

In these formulas (III) and (IV), Y also can represent: 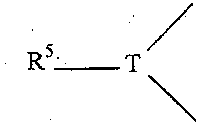 in which R⁵ represents a polyorganosiloxane chain, and T represents a group of formula: 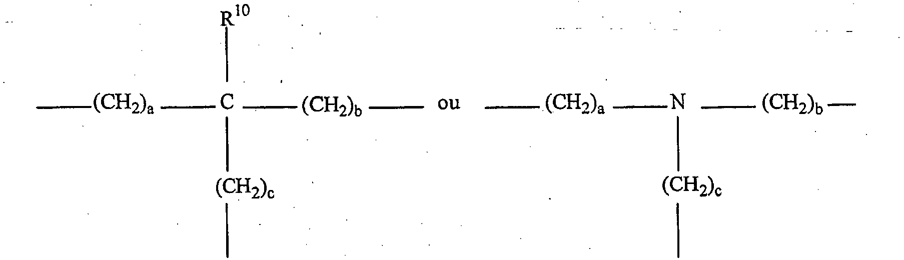 in which a, b and c are, independently, integers ranging from 1 to 10, and R¹⁰ is a hydrogen atom or a group such as those defined for R¹, R², R³ and R⁴.

In formulas (III) and (IV), R¹, R², R³ and R⁴ preferably represent, independently, a linear or branched C₁ to C₄₀ alkyl group, preferably a CH₃, C₂H₅, n-C₃H₇ or isopropyl group, a polyorganosiloxane chain or a phenyl group possibly substituted with one to three methyl or ethyl groups. -

As has been seen previously, the polymer can comprise identical or different moieties of formula (III) or (IV).

Thus the polymer can be a polyamide containing several moieties of formula (III) or (IV) of different lengths, that is, a polyamide corresponding to the formula: 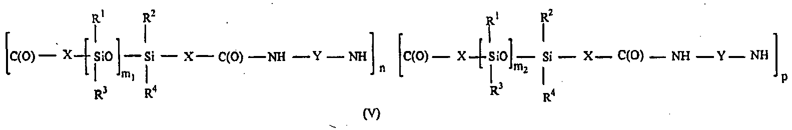 in which X, Y, n, R¹ to R⁴ have the meanings given hereinabove, m₁ and m₂, which are different, are chosen from within the range from 1 to 1000, and p is an integer ranging from 2 to 300.

In this formula, the moieties can be structured to form either a block copolymer, or a random copolymer or an alternating copolymer. In this copolymer, the moieties can be not only of different lengths, but also of different chemical structures, for example having different groups Y. In this case, the copolymer can correspond to the formula: 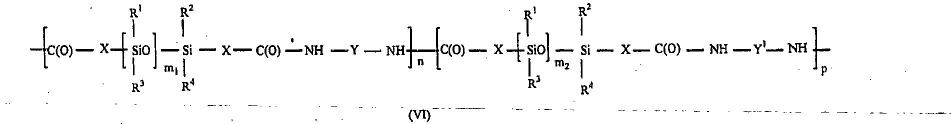 in which R¹ to R⁴, X, Y, m₁, m₂, n and p have the meanings given hereinabove and Y¹ is different from Y but chosen from among the groups defined for Y. As previously, the various moieties can be structured to form either a block copolymer, or a random copolymer or an alternating copolymer.

In this first embodiment of the invention, the gelling agent also can consist of a grafted copolymer. Thus the polyamide with silicone units can be grafted and possibly crosslinked with silicone chains having amide groups. Such polymers can be synthesized with trifunctional amines.

In this case, the copolymer can comprise at least one moiety of formula: 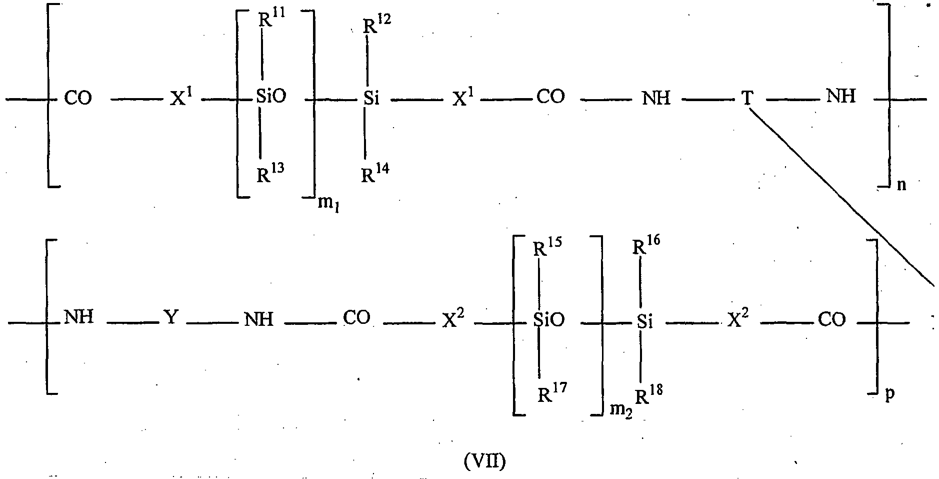 in which X¹ and X², which are identical or different, have the meaning given for X in formula (I), n is such as defined in formula (I), Y and T are such as defined in formula (I), R¹¹ to R¹⁸ are groups chosen from within the same group as R¹ to R⁴, m₁ and m₂ are numbers falling in the range from 1 to 1000, and p is an integer ranging from 2 to 500.

In formula (VII), it is preferred that:
- p is in the range of 1 to 25, better still from 1 to 7,
- R¹¹ to R¹⁸ are methyl groups,
- T corresponds to one of the following formulas: 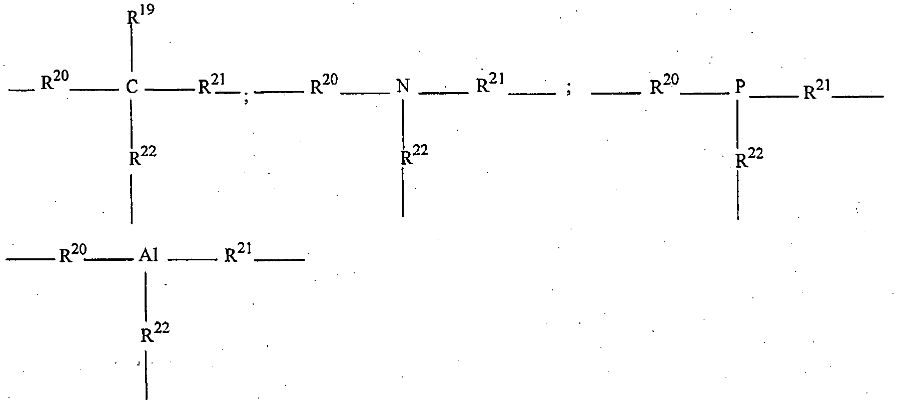 in which R¹⁹ is a hydrogen atom or a group chosen from among the groups defined for R¹ to R⁴, and R²⁰, R²¹ and R²² are, independently, linear or branched alkylene groups, and preferably still, with the formula: 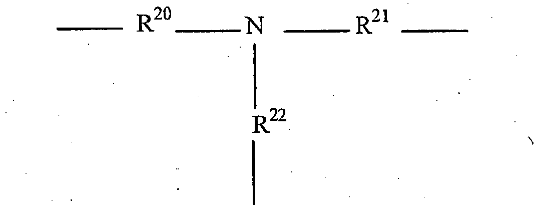 in particular with R²⁰, R²¹ and R²² representing -CH₂-CH₂-,
- m₁ and m₂ are in the range from 15 to 500, and better still from 15 to 45,
- X¹ and X² represent -(CH₂)₁₀-, and
- Y represents -CH₂-.

These polyamides with a grafted silicone moiety of formula (VII) can be copolymerized with polyamide-silicones of formula II to form block copolymers, alternating copolymers or random copolymers. The weight percentage of grafted silicone moieties (VII) in the copolymer can range from 0.5% to 30% by weight.

According to the invention, as has been seen previously, the siloxane units can be in the main chain or backbone of the polymer, but they also can be present in grafted or pendent chains. In the main chain, the siloxane units can be in the form of segments as described hereinabove. In the pendent or grafted chains, the siloxane units can appear individually or in segments.

According to the invention, the preferred siloxane-based polyamides are:
- polyamides of formula (III) in which m is from 15 to 50;
- mixtures of two or more polyamides in which at least one polyamide has a value of m in the range from 15 to 50 and at least one polyamide has a value of m in the range from 30 to 50;
- polymers of formula (V) with m₁ chosen from within the range of 15 to 50 and m₂ chosen from within the range of 30 to 500 with the portion corresponding to m₁ representing 1 to 99% by weight of the total weight of the polyamide and the portion corresponding to m₂ representing 1 to 99% by weight of the total weight of the polyamide;
- mixtures of polyamide of formula (III) combining
   1) 80 to 99% by weight of a polyamide in which n is equal to 2 to 10, in particular 3 to 6, and
   2) 1 to 20% of a polyamide in which n is in the range from 5 to 500, in particular from 6 to 100;
- polyamides corresponding to formula (VI) in which at least one of the groups Y and Y¹ contains at least one hydroxyl substituent;
- polyamides of formula (III) synthesized with at least one portion of an activated diacid (diacid chloride, dianhydride or diester) instead of the diacid;
- polyamides of formula (III) in which X represents - (CH₂)₃- or -(CH₂)₁₀; and
- polyamides of formula (III) in which the polyamides are terminated with a monofunctional chain chosen from within the group consisting of monofunctional amines, monofunctional acids, monofunctional alcohols, including fatty acids, fatty alcohols, and fatty amines, such as, for example, octylamine, octanol, stearic acid and stearyl alcohol.

According to the invention, the ends of the polymer chains can be terminated with:
- a C₁ to C₅₀ alkyl ester group by introducing a C₁ to C₅₀ monoalcohol during synthesis,
- a C₁ to C₅₀ alkylamide group by taking as stopping group a monoacid if the silicone is α,ω-diaminated, or a monoamine if the silicone is an α,w-dicarboxylic acid.
- According to one embodiment variant of the invention, there can be used a copolymer of silicone polyamide and of hydrocarbon polyamide, that is, a copolymer comprising moieties of formula (III) or (IV) and hydrocarbon polyamide moieties. In this case, the polyamide-silicone moieties can be arranged at the ends of the hydrocarbon polyamide.

Polyamide-based gelling agents containing silicones can be produced by silylic amidation of polyamides based on fatty acid dimer. This approach involves the reaction of free acid sites existing on a polyamide as end sites, with oligosiloxane-monoamines and/or oligosiloxane-diamines (amidation reaction), or alternatively with oligosiloxane alcohols or oligosiloxane diols (esterification reaction). The esterification reaction requires the presence of acid catalysts, as is known in the art. It is desirable for the polyamide having free acid sites, used for the amidation or esterification reaction, to have a relatively high number of acid end groups (for example, polyamides having high acid values, for example from 15 to 20).

For the amidation of the free acid sites of the hydrocarbon polyamides, siloxane diamines with 1 to 300, more particularly 2 to 50, and better still 2, 6, 9.5, 12, 13.5, 23 or 31 siloxane groups, can be used for the reaction with hydrocarbon diamides based on fatty acid dimers. Siloxane diamines having 13.5 siloxane groups are preferred, and the best results are obtained with the siloxane-diamine having 13.5 siloxane groups and polyamides containing high values for carboxylic acid end groups.

The reactions may be carried out in xylene to extract the water produced from the solution by azeotropic distillation, or at higher temperatures (about 180 to 200° C) without solvent. Typically, the efficacy of the amidation and the reaction rates decrease when the siloxane diamine is longer, that is, when the number of siloxane groups is higher. Free amine sites can be blocked after the initial amidation reaction of the diaminosiloxanes by reacting them either with an acid siloxane or with an organic acid such as benzoic acid.

For the esterification of the free acid sites on the polyamides, this can be performed in boiling xylene with about 1% by weight, relative to the total weight of the reagents, of para-toluenesulfonic acid as catalyst.

These reactions carried out on the carboxylic acid end groups of the polyamide lead to the incorporation of silicone moieties only at the ends of the polymer chain.

A polyamide-silicone copolymer also can be prepared using a polyamide with free amine groups, by amidation reaction with a siloxane containing an acid group.

There also can be prepared a gelling agent based on a copolymer between a hydrocarbon polyamide and a silicone polyamide, by transamidation of a polyamide having, for example, an ethylene-diamine constituent, with an oligosiloxane-α,w-diamine, at high temperature (for example 200 to 300° C), in order to carry out a transamidation such that the ethylenediamine component of the original polyamide is replaced with the oligosiloxane diamine.

The copolymer of hydrocarbon polyamide and of polyamide-silicone also can be a grafted copolymer comprising a hydrocarbon polyamide backbone with pendent oligosiloxane groups.

This can be obtained, for example:
- by hydrosilylation of unsaturated bonds in polyamides based on fatty acid dimers;
- by silylation of the amide groups of a polyamide; or
- by silylation of unsaturated polyamides by means of an oxidation, that is, by oxidizing the unsaturated groups into alcohols or diols, in order to form hydroxyl groups which are reacted with siloxane carboxylic acids or siloxane alcohols. The olefinic sites of the unsaturated polyamides also can be epoxidized and the epoxy groups then can be reacted with siloxane amines or siloxane alcohols.

According to a second embodiment of the invention, the gelling agent consists of a homopolymer or a copolymer comprising urethane or urea groups.

As previously, the polymer can comprise polyorganosiloxane moieties containing two or more urethane and/or urea groups, either in the backbone of the polymer, or on side chains or as pendent groups.

The polymers comprising at least two urethane and/or urea groups in the backbone may be polymers comprising at least one moiety corresponding to the following formula: 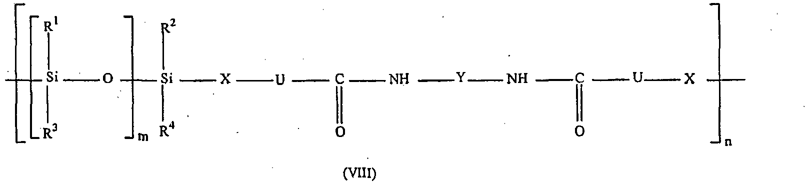 in which R¹ R², R³, R⁴, X, Y, m and n have the meanings given hereinabove for formula (I), and U represents -O- or -NH-, so that: 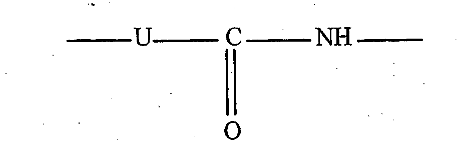 corresponds to a urethane or urea group. In this formula (VIII), Y can be a linear or branched C₁ to C₄₀ alkylene group, possibly substituted with a C₁ to C₁₅ alkyl group or a C₅ to C₁₀ aryl group. Preferably a -(CH₂)₆- group is used.

Y also can represent a C₅ to C₁₂ cycloaliphatic or aromatic group which can be substituted with a C₁ to C₁₅ alkyl group or a C₅ to C₁₀ aryl group, for example a radical chosen from among the methylene-4-4-bicyclohexyl radical, the radical derived from isophorone diisocyanate, 2,4- and 2,6 tolylenes, 1,5 naphthylene, p-phenylene and 4,4'-biphenylenemethane. Generally, it is preferred that Y represent a linear or branched C₁ to C₄₀ alkylene radical, or a C₄ to C₁₂ cycloalkylene radical.

Y also can represent a polyurethane or polyurea sequence corresponding to the condensation of several diisocyanate molecules with one or more molecules of coupling agents of the diol or diamine type. In this case, Y comprises several urethane or urea groups in the alkylene chain.

It can correspond to the formula: 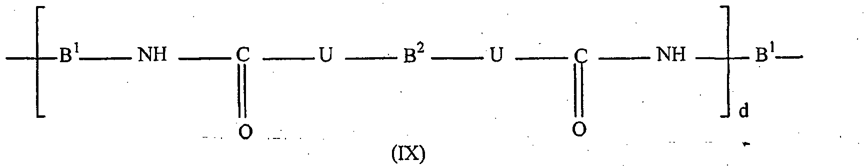 in which B¹ is a group chosen from among the groups given hereinabove for Y, U is -O- or -NH-, and B² is chosen from among:
- linear or branched C₁ to C₄₀ alkylene groups, which possibly can bear an ionizable group such as a carboxylic or sulfonic acid group, or a neutralizable or quaternizable tertiary amine group,
- C₅ to C₁₂ cycloalkylene groups, possibly bearing alkyl, for example one to three methyl or ethyl groups, or alkylene substituents, for example the diol radical: cyclohexanedimethanol,
- phenylene groups which possibly can bear C₁ to C₃ alkyl substituents, and
- groups of formula: 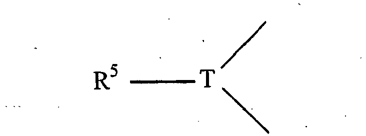 in which T is a hydrocarbon trivalent radical which can contain one or more hetero atoms such as oxygen, sulfur and nitrogen and R⁵ is a polyorganosiloxane chain or linear or branched C₁ to C₅₀ alkyl chain.
   T can represent, for example: 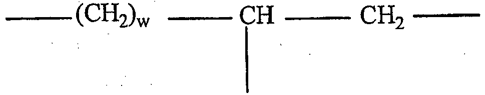 or 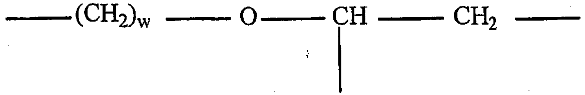 with w being an integer ranging from 1 to 10 and R⁵ being a polyorganosiloxane chain.

When Y is a linear or branched C₁ to C₄₀ alkylene group, the -(CH₂)₂- and -(CH₂)₆- groups are preferred.

In the formula for Y given hereinabove, d can be an integer ranging from 0 to 5, preferably from 0 to 3, preferably still equal to 1 or 2.

B² preferably is a linear or branched C₁ to C₄₀ alkylene group, in particular -(CH₂)₂- or - (CH₂)₆-, or the group: 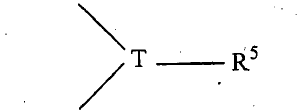 with R⁵ being a polyorganosiloxane chain.

As previously, the polymer constituting the gelling agent can be formed from silicone urethane and/or silicone urea moieties of different length and/or constitution, and be in the form of block, sequenced or random copolymers.

According to the invention, the silicone also can comprise urethane and/or urea groups no longer in the backbone but as side branches.

In this case, the polymer can comprise at least one moiety of formula: 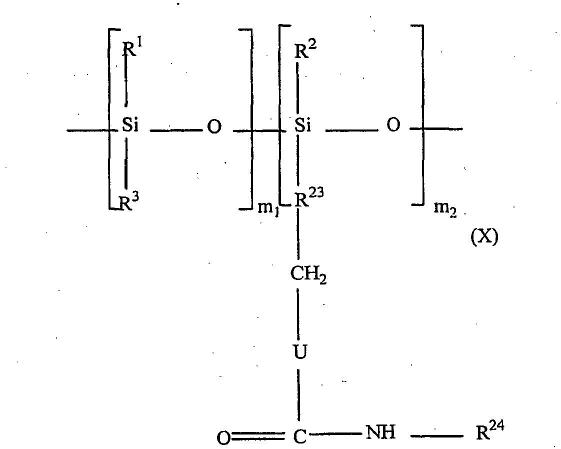 in which R¹, R², R³, m₁ and m₂ have the meanings given hereinabove for formula (I),
- U represents O or NH,
- R²³ represents a C₁ to C₄₀ alkylene group, possibly comprising one or more hetero atoms chosen from among O and N, or a phenylene group, and
- R²⁴ is chosen from among the linear, branched or cyclic, saturated or unsaturated C₁ to C₅₀ alkyl groups, and the phenyl groups possibly substituted with one to three C₁ C₃ alkyl group.

The polymers comprising at least one moiety of formula (X) contain siloxane units and urea or urethane groups, and they can be used as gelling agents in the compositions of the invention.

The siloxane polymers can have a single urea or urethane group by branching or can have branches with two urea or urethane groups, or even contain a mixture of branches with one urea or urethane group and branches with two urea or urethane groups.

They can be obtained from branched polysiloxanes, comprising one or two amino groups by branching, by reacting these polysiloxanes with monoisocyanates.

As examples of starting polymers of this type having amino and diamino branches, there can be cited the polymers corresponding to the following formulas:

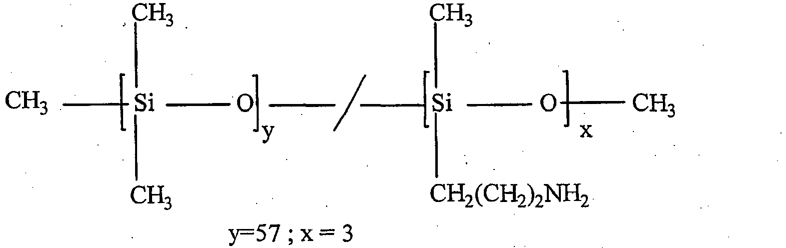

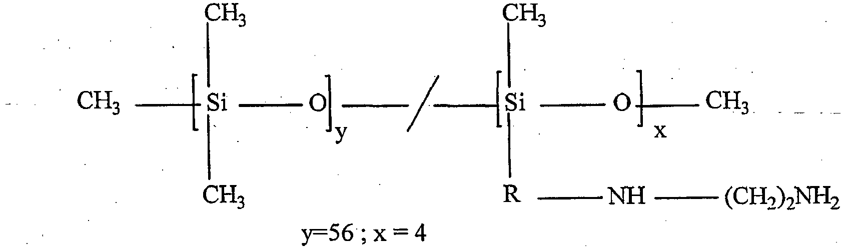

In these formulas, the symbol "/" indicates that the segments can be of different lengths and in a random order, and R represents a linear aliphatic group preferably having 1 to 6 carbon atoms and better still 1 to 3 carbon atoms.

Such polymers with branching can be formed by reacting a siloxane polymer, having at least three amino groups per polymer molecule, with a compound having only one monofunctional group (for example an acid, an isocyanate or isothiocyanate) in order to react this monofunctional group with one of the amino groups and to form groups capable of establishing hydrogen interactions. The amino groups can be on side chains extending from the main chain of the siloxane polymer, so that the groups capable of establishing hydrogen interactions are formed on these side chains, or else the amino groups can be at the ends of the main chain, so that the groups capable of hydrogen interaction will be end groups of the polymer.

As an operating method for forming a polymer containing siloxane units and groups capable of establishing hydrogen interactions, there may be cited the reaction of a siloxane diamine and a diisocyanate in a silicone solvent so as to provide a gel directly. The reaction can be performed in a silicone fluid, the resulting product being dissolved in the silicone fluid, at high temperature, the temperature of the system then being reduced in order to form the gel.

The polymers preferred for incorporation into the compositions according to this invention are siloxane-urea copolymers which are linear and which contain urea groups as groups capable of establishing hydrogen interactions in the backbone of the polymer.

As an illustration of a polysiloxane ending with four urea groups, there may be cited the polymer of formula: 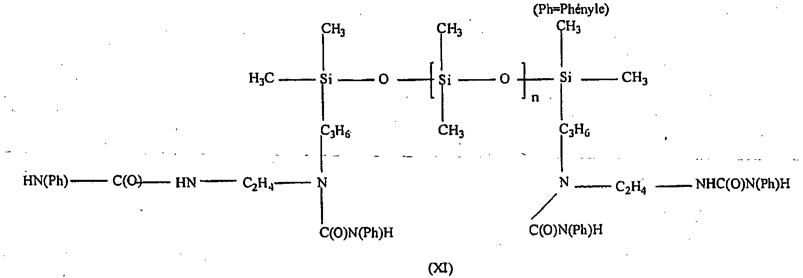 in which Ph is a phenyl group and n is a number from 0 to 300, in particular from 0 to 100, for example 50.

This polymer is obtained by reaction of the following polysiloxane having amino groups: 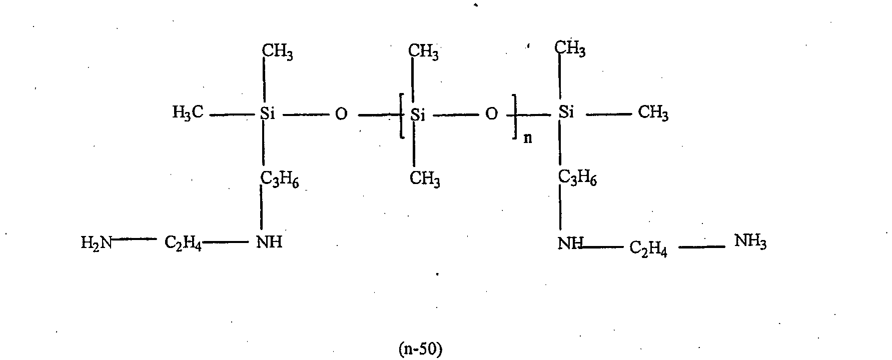 with phenyl isocyanate.

The polymers of formula (VIII) comprising urea or urethane groups in the chain of the silicone polymer can be obtained by reaction between a silicone having α,ω-NH₂ or -OH end groups, of formula: 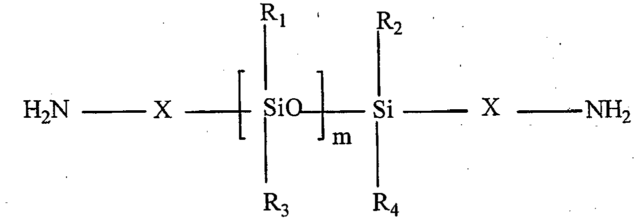 in which m, R¹, R², R³, R⁴ and X are such as defined for formula (I), and a diisocyanate OCN-Y-NCO in which Y has the meaning given in formula (I); and possibly a diol or diamine coupling agent of formula H₂N-B²-NH₂ or HO-B²-OH, in which B² is such as defined in formula (IX).

According to the stoichiometric proportions between the two reagents, diisocyanate and coupling agent, Y can have the formula (IX) with d equal to 0 or d equal to 1 to 5.

As in the case of the silicone polyamides of formula (II) or (III), there may be used in the invention silicone polyurethanes or polyureas having moieties of different length and structure, in particular moieties with lengths differing by the number of silicone units. In this case, the copolymer can correspond, for example, to the formula: 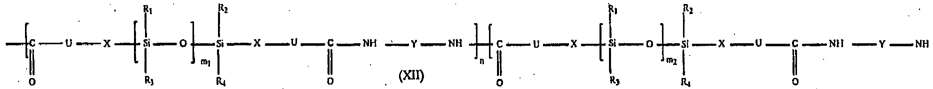 in which R¹, R², R³, R⁴, X, Y and U are such as defined for formula (VIII) and m₁, m₂, n and p are such as defined for formula (V).

Branched silicone polyurethanes or polyureas also may be obtained by using, instead of the diisocyanate OCN-Y-NCO, a triisocyanate of formula:

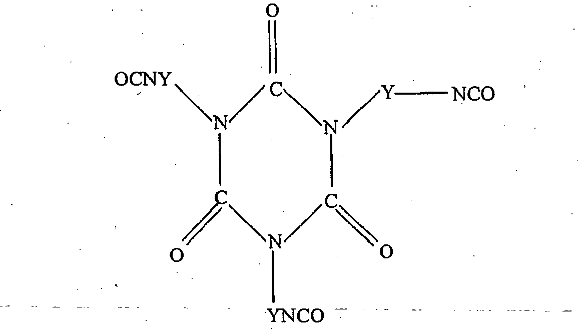

A silicone polyurethane or polyurea having branches comprising an organosiloxane chain with groups capable of establishing hydrogen interactions is obtained in this manner. Such a polymer comprises, for example, a moiety corresponding to the formula: 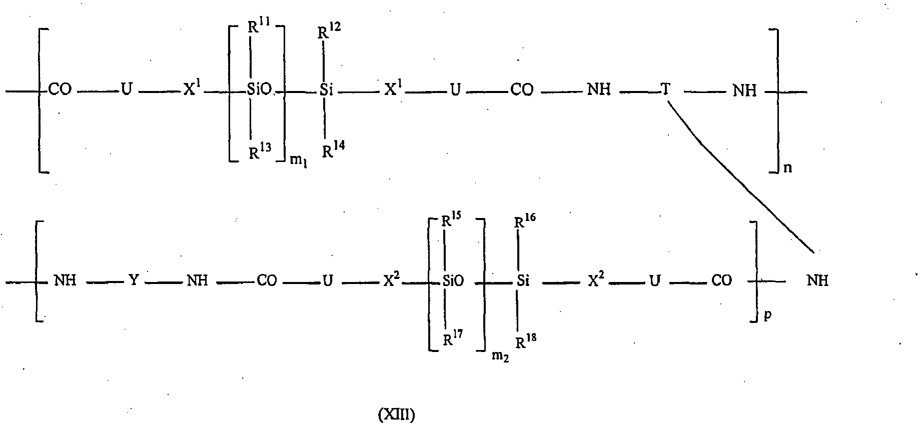 in which X¹ and X², which are identical or different, have the meaning given for X in formula (I), n is such as defined in formula (I), Y and T are such as defined in formula (I), R¹¹ to R¹⁸ are groups chosen from within the same group as R¹ to R⁴, m₁ and m₂ are numbers falling in the range from 1 to 1000, and p is an integer ranging from 2 to 500.

As in the case of the polyamides, this copolymer also can comprise silicone polyurethane moieties without branching.

In this second embodiment of the invention, the preferred siloxane-based polyureas and polyurethanes are:
- polymers of formula (VIII) in which m is from 15 to 50;
- mixtures of two or more polymers in which at least one polymer has a value of m in the range from 15 to 50 and at least one polymer has a value of m in the range from 30 to 50;
- polymers of formula (VIII) with m₁ chosen from within the range from 15 to 50 and m₂ chosen from within the range from 30 to 500 with the portion corresponding to m₁ representing 1 to 99% by weight of the total weight of the polymer and the portion corresponding to m₂ representing 1 to 99% by weight of the total weight of the polymer;
- mixtures of polymer of formula (VIII) combining
   1) 80 to 99% by weight of a polymer in which n is equal to 2 to 10, in particular 3 to 6, and
   2) 1 to 20% of a polymer in which n is in the range from 5 to 500, in particular from 6 to 100,
- copolymers comprising two moieties of formula (VIII) in which at least one of the groups Y contains at least one hydroxyl substituent;
- polymers of formula (VIII) synthesized with at least one portion of an activated diacid (diacid chloride, dianhydride or diester) instead of the diacid;
- polymers of formula (VIII) in which X represents -(CH₂)₃- or -(CH₂)₁₀-; and
- polymers of formula (VIII) in which the polymers are terminated with a monofunctional chain chosen from among,the group consisting of monofunctional amines, monofunctional acids, monofunctional alcohols, including fatty acids, fatty alcohols and fatty amines, such as, for example, octylamine, octanol, stearic acid and stearyl acid.

As in the case of the polyamides, copolymers of silicone polyurethane--or polyurea--and of hydrocarbon polyurethane or polyurea can be used in the invention by performing the reaction for synthesizing the polymer in the presence of an α,ω-difunctional sequence of non-silicone nature, for example a polyester, a polyether or a polyolefin.

As has been seen previously, the gelling agents consisting of homopolymers or copolymers of the invention can have siloxane moieties in the main chain of the polymer and groups capable of establishing hydrogen interactions, either in the main chain of the polymer or at the ends thereof, or on side chains or branches of the main chain. This can correspond to the following five arrangements: 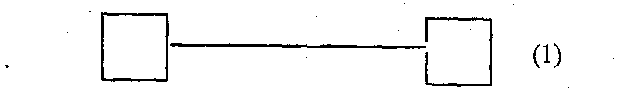 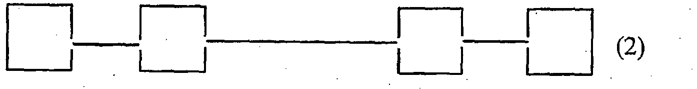 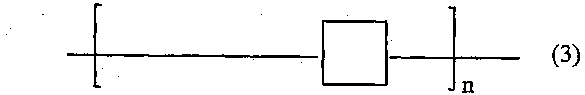 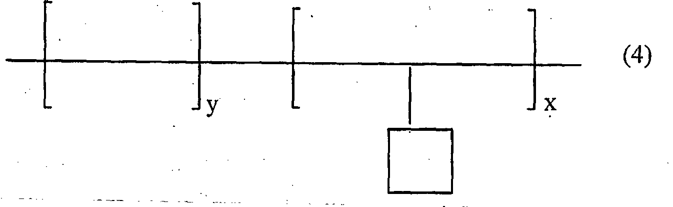 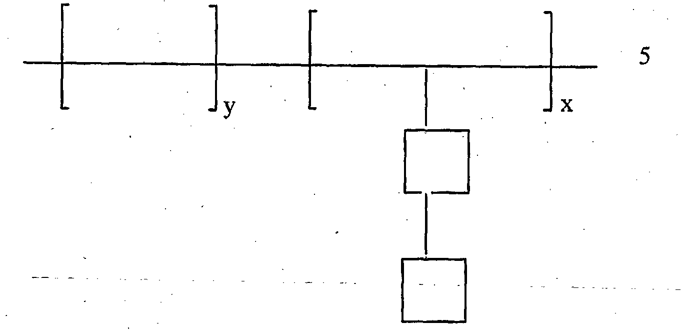 in which the continuous line is the main chain of the siloxane polymer and the squares represent the groups capable of establishing hydrogen interactions.

In case (1), the groups capable of establishing hydrogen interactions are arranged at the ends of the main chain. In case (2), two groups capable of establishing hydrogen interactions are arranged at each of the ends of the main chain.

In case (3), the groups capable of establishing hydrogen interactions are arranged within the main chain in repeating moieties.

In cases (4) and (5), these are copolymers in which the groups capable of establishing hydrogen interactions are arranged on branches of the main chain of a first series of moieties which are copolymerized with moieties not comprising groups capable of establishing hydrogen interactions. The values n, x and y are such that the polymer has the desired - properties as a gelling agent for fatty phases based on silicone oil.

According to the invention, the structuring of the liquid fatty phase of the emulsion containing at least one silicone oil is obtained with the aid of one or more of the polymers mentioned above. According to the invention, these polymers used alone, in the absence of any other gelling, structuring compound, in particular waxes, make it possible to obtain all the effects and advantages of the invention: namely, the obtaining of films having in particular an excellent non-transfer property and a high level of suppleness and flexibility.

As examples of polymers which can be used, there may be cited the silicone polymers obtained in accordance with Examples 1 and 2 of document US-A-5,981,680.

The polymers and copolymers used as gelling agents for the composition of the invention advantageously have a softening point of 65° C to 190° C. They preferably have a softening point ranging from 70 to 130° C and better from 80° C to 105° C. This softening point is lower than that of the known structuring polymers, which facilitates the use of the polymers which are the subject of the invention, makes possible the use of volatile oils and limits the degradation of the liquid fatty phase.

They have good solubility in silicone oils and result in macroscopically homogeneous compositions. They preferably have an average molecular mass from 500 to 200,000, for example from 1,000 to 100,000, preferably from 2,000 to 30,000.

According to the invention, the polymer can be combined with at least one amphiphilic compound liquid at room temperature, with a hydrophilic/lipophilic balance (HLB) value less than 12, in particular ranging from 1 to 7, preferably from 1 to 5, and better from 3 to 5. According to the invention, one or more amphiphilic compounds may be used. These amphiphilic compounds have the purpose of intensifying the structuring properties of the polymer, facilitating the use of the polymer and improving the capacity for depositing of the solid emulsion, for example in the form of a stick.

According to the invention, the emulsion-type composition preferably has a hardness ranging from 20 to 2,000 gf and better from 20 to 900 gf, in particular from 20 to 600 gf and for example from 150 to 450 gf. This hardness can be measured according to a method of penetration of a probe into said composition and in particular with the aid of a texture analyzer (for example TA-TXT2i from Rheo) equipped with an ebonite cylinder 25 mm in height and 8 mm in diameter. The hardness measurement is performed at 20° C at the center of five samples of said composition. The cylinder is introduced into each sample of composition at a pre-speed of 2 mm/s, then at a speed of 0.5 mm/s and finally at a post-speed of 2mm/s, the total displacement being 1 mm. The recorded hardness value is that of the maximum peak. The measurement error is ± 50 gf.

Hardness also can be measured by the so-called cheesewire method, which consists in cutting a lipstick 8.1 or 12.7 mm in diameter and measuring the hardness at 20° C by means of a DFGHS 2 dynamometer from the company Indelco-Chatillon moving at a speed of 100 mm/minute. It is expressed as the shear force (expressed in gramforce) required to cut a stick under these conditions. According to this method, the hardness of a stick composition according to the invention ranges from 30 to 300 gf, preferably from 30 to 250 gf and for example from 30 to 200 gf, when the diameter of the stick is equal to 12.7 mm.

The hardness of the emulsion-type composition according to the invention preferably is such that the composition is self-supporting and can disintegrate easily to form a satisfactory deposit on the skin and/or the lips and/or the phaneric structures. In addition, with this hardness, the composition of the invention has good impact resistance.

According to the invention, the solid emulsion, in particular cast in a dish or possibly in the form of a stick, has the behavior of a deformable, supple elastic solid, imparting a noteworthy elastic softness on application. The cast or stick compositions of the prior art do not have this property of elasticity and suppleness.

The amphiphilic, silicone and non-silicone compound or compounds which can be used in the composition of the invention comprise a lipophilic portion associated with a polar portion, the lipophilic portion comprising a carbon chain having at least 8 carbon atoms, in particular from 18 to 32 carbon atoms, and better from 18 to 28 carbon atoms. The polar portion of this or these amphiphilic compound(s) preferably is the residue of a compound chosen from among the alcohols and polyols having 1 to 12 hydroxyl groups, the polyoxyalkylenes comprising at least two oxyalkylene moieties and having from 0 to 20 oxypropylene moieties and/or from 0 to 20 oxyethylene moieties. In particular, the amphiphilic compound is an ester chosen from among the hydroxystearates; the oleates; the glycerol, sorbitan or methylglucose isostearates; or even C₁₂ to C₂₆ branched fatty alcohols such as octyldodecanol and mixtures thereof. Among these esters, monoesters and mixtures of mono- and diesters are preferred.

The respective levels of structuring silicone polymer and possibly of amphiphilic compound are chosen according to the desired hardness of the gel and in terms of the specific application contemplated. The respective quantities of polymer and possibly of amphiphilic compound are to be such that they make it possible to obtain a cleavable stick. In practice, the quantity of polymer (in active substance) represents from 0.5 to 80% of the total weight of the emulsion, preferably from 2 to 60%, and better from 5 to 40%. The quantity of amphiphilic compound in practice represents from 0.1% to 35% of the total weight of the composition, for example from 1% to 20% and better from 2% to 15%, if it is present.

The gelling polymer/silicone oil(s) mass ratio generally is from 0.1 to 50% by weight.

### Aqueous phase

The composition of the invention, which is an emulsion, also contains an aqueous phase immiscible in the liquid fatty phase containing water possibly thickened or gelled with one or more aqueous-phase thickening or gelling agents and possibly containing compounds miscible with water, such as C₂ to C₇ lower alcohols, polyols having at least two hydroxyl groups and from 2 to 10 carbon atoms such as glycerol, diglycerine, propylene glycol, D-panthenol, sorbitol, polyethylene glycols.

The emulsion according to the invention can be obtained by using a surfactant or a mixture of surfactants the HLB (hydrophilic/lipophilic balance) of which is adapted according to the emulsion.

As a surfactant which can be used in the invention, suitable for obtaining of a W/O emulsion, there may be cited those having an HLB lower than 7 and in particular the fatty acid esters of polyols such as the mono-, di-, tri- or sesquioleates or sorbitol or glycerol stearates, glycerol or polyethylene glycol laurates; alkyl or alkoxy dimethicone copolyols with an alkyl or alcoxy chain pendent or at the end of the silicone backbone having; for example, from 6 to 22 carbon atoms. As a surfactant which can be used in the invention for obtaining of an O/W emulsion, there can be cited those having an HLB in excess of 7, such as the fatty acid esters of polyethylene glycol (polyethylene glycol monostearate or monolaurate); the polyethylene fatty acid (stearate, oleate) esters of sorbitol; the polyoxyethylene alkyl (lauryl, cetyl, stearyl, octyl) ethers and the dimethicone copolyols. In general, any ionic (cationic or anionic) amphoteric surfactant and any non-ionic surfactant, well known to the person skilled in the art, can be used.

### Other additives

The emulsion-type composition of the invention generally constitutes a composition for care and/or treatment and/or makeup for keratinous matter.

In addition, the emulsion of the invention can comprise any ingredient or additive usually used in the field concerned, and in particular those chosen from among the aqueous-phase gelling agents, antioxidants, preservatives, fragrances, electrolytes (cations or polycations), liposoluble polymers or polymers dispersible in the medium, in particular hydrocarbons such as vinyl polylaurate or polyalkylenes, liquid-fatty-phase gelling agents, neutralizers, gums, resins, surfactants such as tri-oleyl phosphate, additional cosmetic or dermatological active substances chosen, for example, from within the group consisting of emollients, moisturizers (glycerin, sodium hyaluronate), vitamins (A, C, D, E, F), essential fatty acids, sunscreens, dispersants such as poly(12-hydroxystearic) acid and mixtures thereof. The composition according to the invention also can contain lipid vesicles of the ionic and/or non-ionic type. These ingredients or additives can be present in the emulsion, usually in a proportion from 0 to 20% of the total weight of the composition, preferably from 0.01 to 20%, and better from 0.1 to 10%.

Of course, the person skilled in the art will make sure to choose the possible additional ingredients and/or the quantity thereof in such manner that the advantageous properties of the composition according to the invention are not, or are not substantially, impaired by the contemplated addition.

The composition according' to the invention may exist in the form of a dermatological or care composition, possibly tinted, for keratinous matter such as the skin, the lips and/or the phaneric structures, in the form of a composition for sun protection, body hygiene or care, in particular in the form of a deodorant or makeup-removal product in stick or case form. It can be used in particular as a care base for the skin, the phaneric structures or the lips (lip balms, protecting the lips against cold and/or sun and/or wind, a cream care product, for example, for the skin, nails or hair).

The composition of the invention also may exist in the form of a colored makeup product for the skin, in particular a foundation, possibly having care or treatment properties, a blusher, a face powder or eye shadow, a concealer product, an eyeliner, a makeup product for the body; makeup for the lips such as a lipstick, a lip gloss or a pencil possibly having care or treatment properties; makeup for the phaneric structures such as the nails, the eyelashes, in particular in the form of a cake mascara, the eyebrows and the hair, particularly in the form of a pencil.

Of course, the composition of the invention should be cosmetically or dermatologically acceptable, that is, contain a non-toxic physiologically acceptable medium able to be applied to the skin, phaneric structures or lips of humans. In the meaning of the invention, cosmetically acceptable is understood as a composition with pleasant appearance, odor, taste and feel.

The composition advantageously contains at least one cosmetic active substance and/or one dermatological active substance and/or at least one coloring agent. By virtue of the use of at least one polymer with a weight-average molecular mass ranging from 1000 to 30,000, such as defined previously in an emulsion, a trapping of the active substances and the coloring agents present in the composition is obtained, making it possible to keep them where they have been applied, that is, the lips, skin or phaneric structures.

The said cosmetic and/or dermatological active substance can be chosen in particular from among essential oils, vitamins, moisturizers, filters, healing substances and ceramides.

In the case of makeup compositions, solid hydrophobic or hydrophilic particles can constitute the pigment(s) making it possible to make up the skin, lips and/or phaneric structures.

In addition, the makeup or care compositions according to the invention can comprise at least 10% by mass of a non-volatile oil (silicone or non-silicone) with a view to obtaining a comfortable and non-pulling product.

According to the invention, the composition furthermore can contain a coloring agent which can be chosen from among the lipophilic dyes, hydrophilic dyes, pigments and nacres usually used in cosmetic or dermatological compositions, and mixtures thereof. This coloring agent generally is present in a proportion from 0.01 to 50% of the total weight of the composition, preferably from 5 to 30%, if it is present.

The liposoluble dyes are, for example, Sudan Red, D&C Red 17, D&C Green 6, β-carotene, soybean oil, Sudan Brown, D&C Yellow 11, D&C Violet 2, D&C Orange 5, quinoline yellow or annatto. The hydrophilic dyes are, in particular, beetroot juice and methylene blue. The soluble dyes can represent from 0 to 20% of the weight of the composition and better from 0.1 to 6% (if present).

The pigments and the nacreous pigments or nacres, as well as their proportions, already have been described hereinabove.

It is to be noted that the emulsion-type compositions according to the invention contain, in comparison with the emulsions of the prior art, very small, even zero, quantities of wax and that, because of this, all the drawbacks connected with high proportions of wax are avoided for the compositions of the invention.

The emulsion-type composition according to the invention can be manufactured by known processes generally used in the cosmetic or dermatological field. It can be manufactured by the process which consists in heating the polymer at least to its softening point, adding the oil(s) and, if necessary, the amphiphilic compound or compounds thereto, then mixing the whole until a clear solution is obtained. To the mixture obtained, there then are added the coloring agents and/or solid particles with stirring, then, after lowering of the temperature, the volatile compound or compounds, active substances, fragrance, then the aqueous phase. The whole then is homogenized. The homogeneous mixture obtained then can be cast in a suitable mold such as a lipstick mold or directly into packaging articles (case or dish or jar of lip gloss, in particular).

The invention also has as a purpose a cosmetic solid emulsion as defined above, for making-up of the skin, the lips and/or phaneric structures containing at least one pigment in sufficient quantity for making up the skin, lips and/or phaneric structures, an aqueous phase, and a liquid fatty phase dispersed one within the other, said liquid fatty phase comprising at least one silicone oil, structured with at least one polymer (homopolymer or copolymer) with a weight-average molecular mass ranging from 500 to 500,000, containing at least one moiety comprising:
- at least one polyorganosiloxane group, composed of 1 to 1000 organosiloxane units in the chain of the moiety or in the form of a graft, and
- at least two groups capable of establishing hydrogen interactions chosen from among ester, amide, sulfonamide, carbamate, thiocarbamate, urea, thiourea, oxamido, guanidino, biguanidino groups, and combinations thereof, on condition that at least one group is other than an ester group,
the polymer being solid at room temperature and soluble in the liquid fatty phase at a temperature of 25 to 250EC, the liquid fatty phase consisting in whole or in part of silicone oil(s),

The pigment, the aqueous phase, the liquid fatty phase and the polymer forming a physiologically acceptable medium. The emulsion advantageously exists in the form of a solid.

This emulsion-type makeup composition preferably is self-supporting.

The liquid fatty phase preferably constitutes the continuous or external phase of the composition.

The invention also relates to a cosmetic solid emulsion as defined above, for lipstick, containing at least one pigment in sufficient quantity for making up the lips, an aqueous phase, and a liquid fatty phase dispersed one within the other, said liquid fatty phase comprising at least one silicone oil, structured with at least one polymer (homopolymer or copolymer) with a weight-average molecular mass ranging from 500 to 500,000, containing at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among ester, amide, sulfonamide, carbamate, thiocarbamate, urea, thiourea, oxamido, guanidino, biguanidino groups, and combinations thereof, on condition that at least one group is other than an ester group,
the polymer being solid at room temperature and soluble in the liquid fatty phase at a temperature of 25 to 250°C, the liquid fatty phase consisting in whole or in part of silicone oil(s),

The pigment, the aqueous phase, the liquid fatty phase and the polymer forming a physiologically acceptable medium. In particular, the emulsion exists in the form of a solid.

The emulsion of the invention also can exist in the form of a cake mascara, an eyeliner, a foundation, a lipstick, a blusher, a deodorant or makeup-removal product, a makeup product for the body, an eye shadow or face powder, a concealer, a sun-protection product, a product for care of the face or body.

The invention also has as a purpose a makeup stick constituted of a solid emulsion as defined above, for the skin, lips and/or phaneric structures, and in particular the lips, containing at least one pigment in sufficient quantity for making up the skin, lips and/or phaneric structures, an aqueous phase dispersed in a continuous liquid fatty phase, said liquid fatty phase comprising at least one volatile silicone oil, and being structured with at least one polymer (homopolymer or copolymer) with a weight-average molecular mass ranging from 500 to 500,000, containing at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among ester, amide, sulfonamide, carbamate, thiocarbamate, urea, thiourea, oxamido, guanidino, biguanidino groups, and combinations thereof, on condition that at least one group is other than an ester group,
   the polymer being solid at room temperature and soluble in the liquid fatty phase at a temperature of 25 to 250°C,
   the liquid fatty phase consisting in whole or in part of silicone oil(s), the pigment, the aqueous phase, the fatty phase and the polymer forming a physiologically acceptable medium.

The invention relates to a cosmetic process for care and/or makeup and/or treatment of keratinous matter in humans, consisting in the application to the keratinous matter of a cosmetic solid emulsion according to the invention, as defined above.

Diclosed herewith is also the use of a sufficient quantity of at least one polymer (homopolymer or copolymer) with a weight-average molecular mass ranging from 500 to 500,000, containing at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among ester, amide, sulfonamide, carbamate, thiocarbamate, urea, thiourea, oxamido, guanidino, biguanidino groups, and combinations thereof, on condition that at least one group is other than an ester group,
   the polymer being solid at room temperature and soluble in the liquid fatty phase at a temperature of 25 to 250°C, in a cosmetic emulsion or for the manufacture of a physiologically acceptable emulsion, containing an aqueous phase, a liquid fatty phase dispersed one within the other, sad liquid fatty phase comprising at least one silicone oil, the liquid fatty phase consisting in whole or in part of silicone oil(s), for structuring said emulsion in the form of a self-supporting solid with a hardness ranging from 20 to 2000 gf and preferably from 20 to 900 gf and better from 20 to 600 gf.

Further, it is also diclosed the use of a liquid fatty phase comprising at least one silicone oil, basically structured with a sufficient quantity of at least one polymer (homopolymer or copolymer) with a weight-average molecular mass ranging from 500 to 500-,000, containing at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among ester, amide, sulfonamide, carbamate, thiocarbamate, urea, thiourea, oxamido, guanidino, biguanidino groups, and combinations thereof, on condition that at least one group is other than an ester group,
the polymer being solid at room temperature and soluble in the liquid fatty phase at a temperature of 25 to 250°C,
the liquid fatty phase consisting in whole or in part of volatile oil(s) with a vapor pressure in excess of 0.02 mm of mercury (Hg) in a cosmetic emulsion or for the manufacture of a physiologically acceptable emulsion, glossy and/or non-migrating and/or without transfer. Said emulsion containing an aqueous phase and the liquid fatty phase dispersed one within the other and said emulsion being rigid, glossy, and/or non-migrating, and/or without transfer.

Further disclosed is also the use of a sufficient quantity of at least one polymer (homopolymer or copolymer) with a weight-average molecular mass ranging from 500 to 500,000, containing at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among ester, amide, sulfonamide, carbamate, thiocarbamate, urea, thiourea, oxamido, guanidino, biguanidino groups, and combinations thereof, on condition that at least one group is other than an ester group,
   the polymer being solid at room temperature and soluble in the liquid fatty phase at a temperature of 25 to 250°C,
   in a cosmetic emulsion or for the manufacture of a physiologically acceptable emulsion, said emulsion containing an aqueous phase and a liquid fatty phase dispersed one within the other, said liquid fatty phase comprising at least one silicone oil as an agent for structuring said composition in the form of a self-supporting solid.

The use of a liquid fatty phase is disclosed herein, comprising at least one silicone oil, basically structured with a sufficient quantity of at least one polymer (homopolymer or copolymer) with a weight-average molecular mass ranging from 500 to 500,000, containing at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among ester, amide, sulfonamide, carbamate, thiocarbamate, urea, thiourea, oxamido, guanidino, biguanidino groups, and combinations thereof, on condition that at least one group is other than an ester group,
   the polymer being solid at room temperature and soluble in the liquid fatty phase at a temperature of 25 to 250°C,
   the liquid fatty phase consisting in whole or in part of volatile oil(s) having a flash point equal to or in excess of 40°C, in a cosmetic emulsion or for the manufacture of a physiologically acceptable emulsion comprising an aqueous phase and said liquid fatty phase dispersed one within the other, as an agent for improving the properties of non-transfer and/or suppleness and flexibility of a deposit obtained from said emulsion.

According to an advantageous characteristic of these uses, the emulsion-type composition has a hardness from 20 to 2000 gf, preferably from 20 to 900 gf, and better from 20 to 600 gf, measured with the aid of the texture analyzer.

Finally, the invention concerns a cosmetic process for improving the properties of non-transfer and/or suppleness and/or flexibility and/or staying power of a deposit obtained from a cosmetic solid emulsion as defined above, containing an aqueous phase and a liquid fatty phase dispersed one within the other, said liquid fatty phase comprising at least one volatile silicone oil, and consisting in structuring said fatty phase with a sufficient quantity of at least one polymer (homopolymer or copolymer) with a weight-average molecular mass ranging from 500 to 500,000, consisting of at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among ester, amide, sulfonamide, carbamate, thiocarbamate, urea, thiourea, oxamido, guanidino, biguanidino groups, and combinations thereof, on condition that at least one group is other than an ester group,
   the polymer being solid at room temperature and soluble in the liquid fatty phase at a temperature of 25 to 250° C,
   the liquid fatty phase consisting in whole or in part of silicone oil(s).

### Detailed presentation of the invention

The invention is illustrated in greater detail in the following examples of formulation of makeup, in particular foundation. The quantities are given as % by mass. The chemical compounds are given mainly as INCI or CTFA names, well known to formulators of cosmetics.

### Example 1: foundation without transfer

| Phase | INCI Name | % |
|---|---|---|
| A | Cyclopentasiloxane (and) dimethicone copolyol | 8.0 |
| | Polyglyceryl-4 isostearate (and) hexyl laurate (and cetyl PEG/PPG-10/1 dimethicone | 3.5 |
| | Treated pigments | 9.9 |
| B1 | Cyclopentasiloxane (volatile oil) | 26.1 |
| | Polysiloxane/polyamide (PASi) | 3.0 |
| B2 | Polytrap/cyclopentasiloxane (filler) | 1.0 |
| | MMA crosspolymer (filler) | 4.0 |
| | Nylon-12 (filler) | 1.0 |
| B3 | Preservative | 0.4 |
| | Disteardimonium Hectorite | 0.6 |
| | Propylene carbonate | 0.2 |
| C | Water | 40.0 |
| | Magnesium sulfate | 1.0 |
| | Preservatives | 0.7 |
| | Non-ionic emulsifier | 0.5 |
| | Total | 100.0 |

The foundation is obtained by heating phase B1 until softening of this phase; adding phase B2 with stirring, then phase B3; adding phase A at a temperature 20° C below the preceding temperature, then adding phase C under stirring.

The product obtained in this way has, because of the incorporation into the liquid fatty phase of- a polysiloxane (PS) polyamide (PA) polymer, according to the invention, excellent non-transfer properties (demonstrated by the non-deposition of foundation on a small collar placed around the neck of made-up testers for several minutes).

The use of the PS-PA polymer alone, in the fatty phase, makes it possible to obtain these non-transfer properties. In addition, this foundation has the following properties: non-stickiness, freshness, comfort.

The foundation makes possible a smooth, easy application with an excellent slip ("smooth application with excellent slip and cushion").

The deposit also has an excellent resistance to water.

### Example 2: foundation without transfer

| Phase | INCI Name | % |
|---|---|---|
| A | Cyclopentasiloxane (and) dimethicone copolyol | 8.0 |
| | Polyglyceryl-4 isostearate (and) hexyl laurate (and) cetyl (PEG/PPG-10/1 dimethicone | 3.5 |
| | Pigments | 9.9 |
| B1 | Cyclopentasiloxane | 16.1 |
| | Polysiloxane/polyamide PASi (MW : 14 × 10⁴, DP 15) | 1.0 |
| | Silicone-acrylates | 12.0 |
| B2 | Polytrap/cyclopentasiloxane (filler) | 1.0 |
| | MMA Crosspolymer (filler) | 4.0 |
| | Nylon-12 (filler) | 1.0 |
| B3 | Preservatives | 0.4 |
| | Disteardimoniun Hectorite (gelling agent) | 0.6 |
| | Propylene Carbonate | 0.2 |
| C | Water | 40.0 |
| | Magnesium sulfate | 1.0 |
| | Methylparaben (preservative) | 0.7 |
| | Non-ionic emulsifier | 0.5 |
| | Total | 100.0 |

This foundation is obtained in the following manner: heating of the silicone polyamide in the non-volatile oils until obtaining of a liquid mixture, addition of pigments, fillers, gelling agent, surfactants, then volatiles at 20° C below the softening point of the polymer, addition of the aqueous phase, preservatives, and magnesium sulfate, then homogenization of the whole. The product obtained then is poured into a suitable container, of the foundation-case type.

The product obtained in this manner has, because of the incorporation into the liquid fatty phase of the combination of a polysiloxane (PS)/polyamide (PA) polymer and silicone acrylates, excellent non-transfer properties (demonstrated by the non-deposition of foundation on a small collar placed around the neck of made-up testers for several minutes). In addition, this foundation has the following properties: non-stickiness, flexibility, comfort, freshness. It makes possible a good application ("good application with cushion") and the deposit has an excellent resistance to water.

### Example 3 : foundation without transfer

| Phase | INCI Name | % |
|---|---|---|
| A | Ethylhexyl Methoxycinnamate | 4.0 |
| | Cyclopentasiloxane (and) dimethicone copolyol | 8.0 |
| | Cyclopentasiloxane (and) diphenyl dimethicone | 2.0 |
| | Pigments | 9.9 |
| B | Cyclopentasiloxane | 18.0 |
| | Polysiloxane/polyamide | 3.0 |
| | Polyglyceryl-4-isostearate (and) hexyl laurate (and) cetyl PEG/PPG-10/1 dimethicone | 3.5 |
| | Preservative | 0.2 |
| C | MMA crosspolymer (filler) | 4.5 |
| | Polytrap in cyclopentasiloxane (filler) | 0.9 |
| | Silica (filler) | 0.64 |
| D | Water | 32.16 |
| | Butylene glycol (moisturizer) | 10.0 |
| | Magnesium sulfate | 1.0 |
| | Methylparaben (preservative) | 0.3 |
| | Non-ionic emulsifier | 0.5 |
| E | Water | 1.0 |
| | Preservative | 0.3 |
| | TOTAL | 100.0 |

This foundation is obtained in the following manner.

To begin, phase A is prepared by mixing the ingredients well and grinding them with a SILVERSON homogenizer at a speed of 6000 rpm. Separately, the phase B ingredients are heated to 80 to 85° C, with stirring for 10 to 15 minutes, or until dissolution of the polysiloxane/polyamide. Phases A and B then are combined in the main beaker and mixed well at the temperature of 60 to 65° C. Phase C is added to the main beaker and it is stirred until obtaining of a uniform mixture.

Phase D is heated to 65 to 70° C in a separate side beaker. Emulsification is carried out by adding phase D to the main beaker with the aid of a homogenizer at medium/high speed. The product is cooled to 40 to 45° C, then phase E is added slowly with thorough stirring. The product is cooled to room temperature, by means of a paddle stirrer.

The product obtained in this manner has, because of the incorporation into the liquid phase of a polysiloxane/polyamide polymer (PASi), excellent cosmetic properties, in particular an easy, flowing application ("nice application with cushion"), a good resistance to transfer after drying, a very good resistance to water, a high level of comfort ("comfortable wear").

### Example 4 : foundation without transfer

| Phase | INCI Name | % |
|---|---|---|
| A | Ethylhexyl Methoxycinnamate | 4.0 |
| | Cyclopentasiloxane (and) dimethicone copolyol | 8.0 |
| | Cyclopentasiloxane (and) diphenyl dimethicone | 2.0 |
| | Pigments | 9.9 |
| B | Cyclopentasiloxane | 18.0 |
| | Polysiloxane/polyamide | 3.0 |
| | Polyglyceryl-4-isostearate (and) hexyl laurate (and) cetyl PEG/PPG-10/1 dimethicone | 3.5 |
| | Propylparaben | 0.2 |
| C | MMA crosspolymer | 4.5 |
| | Polytrap/cyclopentasiloxane | 0.9 |
| | Silica | 0.64 |
| D | Water | 42.16 |
| | Magnesium sulfate | 1.0 |
| | Preservative | 0.3 |
| | Non-ionic emulsifier | 0.5 |
| E | Water | 1.0 |
| | Preservative | 0.3 |
| | TOTAL | 100.0 |

The foundation is obtained in a manner comparable to that described previously in example 3.

The product obtained in this manner has, because of the incorporation into the fatty phase of a polysiloxane/polyamide polymer, excellent cosmetic properties, in particular of non-transfer and staying power, and properties of "comfortable wear, "nice application with cushion" and it is light during application - "feel light during application."

### REFERENCES

[1] EP-A-1 068 856
[2] WO-A-01/97758
[3] US-A-5 874 069
[4] US-A-5 919 441
[5] US-A-6 051 216
[6] WO-A-02/17870
[7] WO-A-02/17871
[8] EP-A-1 177 784
[9] US-A-5 412 004
[10] EP-A-1 048 686
[11] US-A-5 981 680
[12] WO-A-99/06473
[14] US-A-6 353 076.

## Claims

1. Cosmetic solid emulsion for care and/or makeup, **characterized in that** (i) it does not flow under its own weight at room temperature and atmospheric pressure, (ii) it comprises an aqueous phase and a liquid fatty phase dispersed one within the other, said liquid fatty phase comprising at least one volatile silicone oil representing from 20 to 50 % by weight of the total weight of the emulsion and being chosen from within the group consisting of the following compounds: octyltrimethicone, hexyltrimethicone, decamethyl cyclopentasiloxane D5, octamethyl cyclotetrasiloxane D4, dodecamethyl cyclohexasiloxane D6, heptamethyl octyltrisiloxane, decamethyl tetrasiloxane, dodecamethyl pentasiloxane, 1.5 cSt polydimethylsiloxane, 2 cSt polydimethylsiloxane, 3 cSt polymethylsiloxane, 5 cSt polydimethylsiloxane, wherein cSt is a viscosity unit equivalent to 10⁻⁶m²·s⁻¹, and mixtures thereof, and (iii) said liquid fatty phase is structured with at least one gelling polymer (homopolymer or copolymer) with a weight-average molecular mass ranging from 500 to 500,000, containing at least one moiety comprising:
- at least one polyorganosiloxane group, composed of 1 to 1000 organosiloxane units in the chain of the moiety or in the form of a graft, and
- at least two groups capable of establishing hydrogen interactions, chosen from among the ester, amide, sulfonamide, carbamate, thiocarbamate, urea, thiourea, oxamido, guanidino, biguanidino groups, and combinations thereof, on condition that at least one of the groups is other than an ester group,
the polymer being solid at room temperature and soluble in the liquid fatty phase at a temperature of 25 to 250°C, the aqueous phase, the liquid fatty phase and the gelling polymer forming a physiologically acceptable medium and, **characterized in that** it comprises a coloring agent.

2. Emulsion according to claim 1, **characterized in that** the fatty phase consists solely of volatile silicone oil(s) preferably having a flash point equal to or in excess of 40°C and/or a viscosity less than 8 cSt, wherein cSt is a viscosity unit equivalent to 20⁻⁶m²·s⁻¹.

3. Emulsion according to Claim 1 or 2, **characterized in that** the volatile oil has a flash point in excess of 60°C.

4. Emulsion according to anyone one of Claims 1 to 3 **characterized in that** the volatile oil has a flash point equal to or less than 135°C.

5. Emulsion according to anyone of Claims 1 to 4, **characterized in that** it comprises in addition solid particles chosen from among the fillers; pigments, nacreous or otherwise; and mixtures thereof.

6. Emulsion according to claim 5, **characterized in that** the solid particles are hydrophobic particles.

7. Emulsion according to claim 6, **characterized in that** the solid particles are hydrophilic particles, coated in a film of hydrophobic compound.

8. Emulsion according to claim 5, **characterized in that** the solid particles are hydrophilic particles and the emulsion further comprises at least one amphiphilic silicone.

9. Emulsion according to claim 6, **characterized in that** the solid particles consist of powders or fibers of hydrophobic polymers or copolymers.

10. Emulsion according to Claim 5 to 9, **characterized in that** the particles are inorganic pigments chosen from among the zinc oxides, iron oxides, titanium oxides and mixtures thereof.

11. Emulsion according to anyone of the preceding claims, **characterized in that** the polymer comprises at least one moiety of formula (III) or (IV): 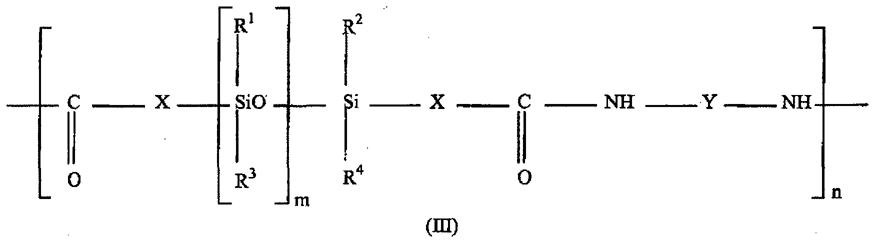 or 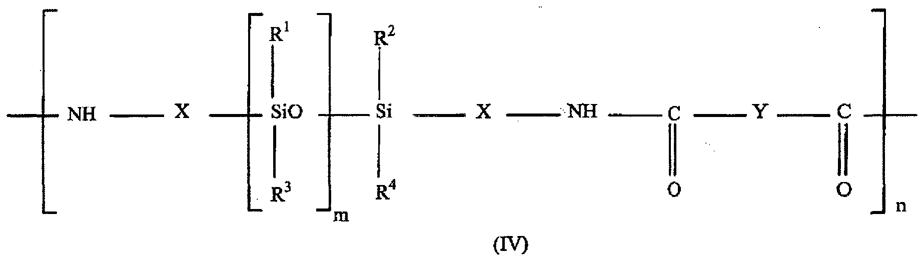 in which 1) R¹, R², R³ and R⁴, identical or different,
represent a group chosen from among:
- linear, branched or cyclic, saturated or unsaturated, C₁ to C₄₀ hydrocarbon groups, which can contain in their chain one or more oxygen, sulfur and/or nitrogen atoms, and which can be partly or totally substituted with fluorine atoms,
- C₆ to C₁₀ aryl groups, possibly substituted with one or more C₁ to C₄ alkyl groups, polyorganosiloxane chains containing or not containing one or more oxygen, sulfur and/or nitrogen atoms;
2) the groups X, identical or different, represent a linear or branched C₁ to C₃₀ alkylenediyl group, which can contain in its chain one or more oxygen and/or nitrogen atoms;
3) Y is a saturated or unsaturated, C₁ to C₅₀ linear or branched divalent alkylene, arylene, cycloalkylene, alkylarylene or arylalkylene group, which can comprise one or more oxygen, sulfur and/or nitrogen atoms, and/or bear as substituent one of the following atoms or groups of atoms: fluorine, hydroxy, C₃ to C₈ cycloalkyl, C₁ to C₄₀ alkyl, C₅ to C₁₀ aryl, phenyl possibly substituted with 1 to 3 C₁ to C₃ alkyl groups, C₁ to C₃ hydroxyalkyl and C₁ to C₆ aminoalkyl, or 4) Y represents a group corresponding to the formula: 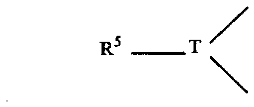
in which - T represents a linear or branched, saturated or unsaturated, C₃ to C₂₄ trivalent or tetravalent hydrocarbon group, possibly substituted with a polyorganosiloxane chain, which can contain one or more atoms chosen from among O, N and S, or T represents a trivalent atom chosen from among N, P and Al, and
- R⁵ represents a linear or branched C₁ to C₅₀ alkyl group, or a polyorganosiloxane chain, which can comprise one or more ester, amide, urethane, thiocarbamate, urea, thiourea and/or sulfonamide groups which can be linked or not linked to another chain of the polymer,
5) n is an integer ranging from 2 to 500, preferably from 2 to 200, and m is an integer ranging from 1 to 1000, preferably from 1 to 700, and better still from 6 to 200.

12. Emulsion according to Claim 11, **characterized in that** X and/or Y represent an alkylene group containing in its alkylene portion at least one of the following elements:
1) 1 to 5 amide, urea or carbamate groups.
2) a C₅ or C₆ cycloalkyl group, and
3) a phenylene group possibly substituted with 1 to 3, identical or different, C₁ to C₃ alkyl groups, and/or substituted with at least one element chosen from among the group consisting of:
- a hydroxy group,
- a C₃ to C₈ cycloalkyl group,
- one to three C₁ to C₄₀ alkyl groups,
a phenyl group possibly substituted with one to three C₁ to C₃ alkyl groups,
- a C₁ to C₃ hydroxyalkyl group, and
- a C₁ to C₆ aminoalkyl group.

13. Emulsion according to Claim 11, **characterized in that** Y represents: 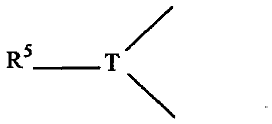 in which R⁵ represents a polyorganosiloxane chain, and T represents a group of formula: 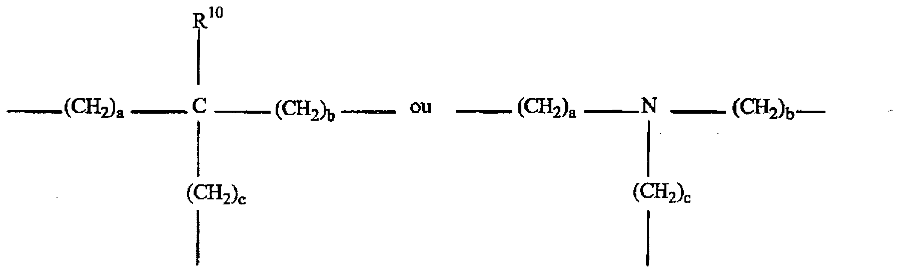 in which a, b and c are, independently, integers ranging from 1 to 10, and R¹⁰ is a hydrogen atom or a group such as those defined for R¹, R², R³ and R⁴, in claim 17.

14. Emulsion according to Claim 11, **characterized in that** R¹, R², R³ and R⁴ represent, independently, a linear or branched, C₁ to C₄₀ alkyl group, preferably a CH₃, C₂H₅, n-C₃H₇ or isopropyl group, a polyorganosiloxane chain or a phenyl group possibly substituted with one to three methyl or ethyl groups.

15. Emulsion according to anyone of Claims 1 to 10, **characterized in that** the gelling polymer comprises at least one moiety of formula: 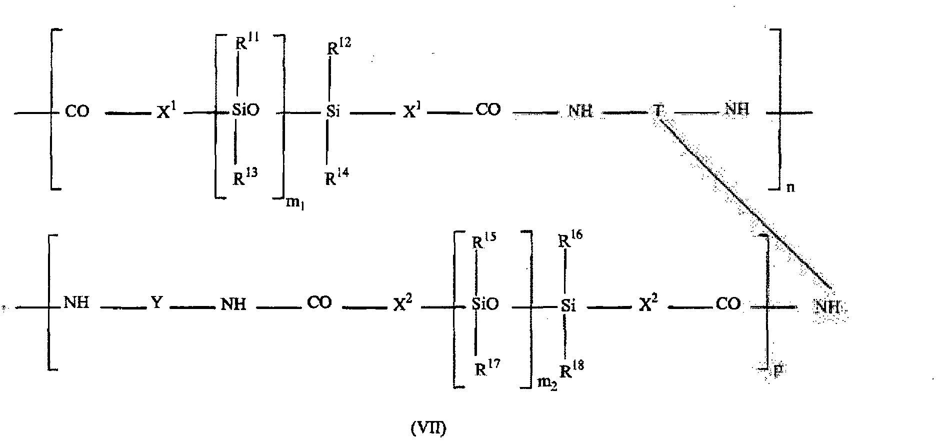 in which X¹ and X² which are identical or different, have the meaning given for X in claim 11, n, Y and T are such as defined in claim 11, R¹¹ to R¹⁸ are groups chosen from within the same group as R¹ to R⁴ of claim 11, m₁ and m₂ are numbers falling in the range from 1 to 1000, and p is an integer ranging from 2 to 500.

16. Emulsion according to claim 15, **characterized in that**:
- p is in the range from 1 to 25, better still from 1 to 7,
- R¹¹ to R¹⁸ are methyl groups,
- T corresponds to one of the following formulas: 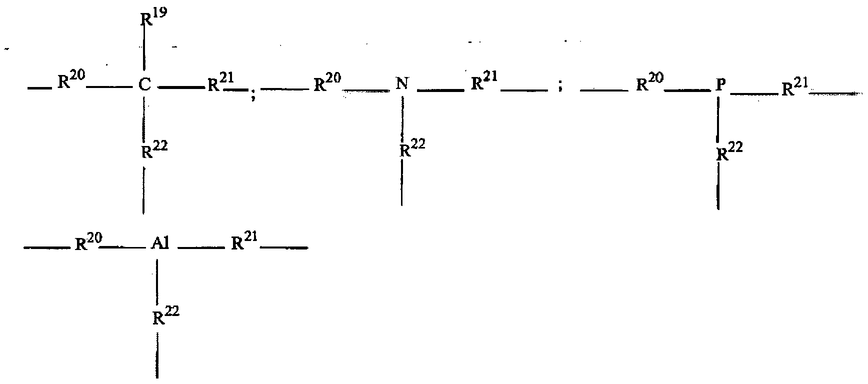 in which R¹⁹ is a hydrogen atom or a group chosen from among the groups defined for R¹ to R4, and R²⁰, R²¹ and R²² are, independently, linear or branched alkylene groups, and more preferably to the formula: 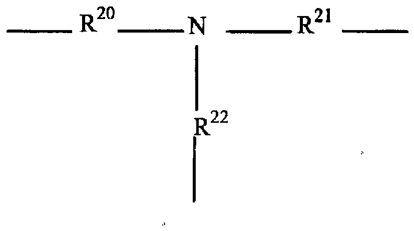 in particular with R²⁰ R²¹ and R²² representing -CH₂-CH₂-,
- m₁ and m₂ are in the range from 15 to 500, and better still from 15 to 45,
- X¹ and X² represent -(CH₂)₁₀-, and
- T represents -CH₂-.

17. Emulsion according to anyone of Claims 1 to 10, **characterized in that** the gelling polymer comprises at least one moiety corresponding to the following formula: 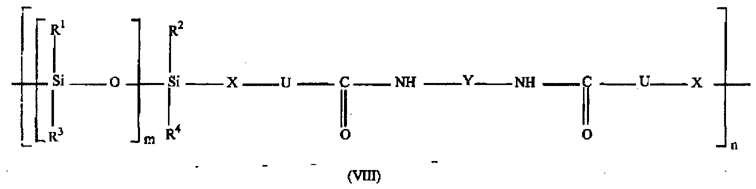 in which R¹, R², R³, R⁴, X, Y, m and n have the meanings given hereinabove for formula (I) in claim 11, and U represents -O- or -NH- or
Y represents a C₅ to C₁₂ cycloaliphatic or aromatic group which can be substituted with a C₁ to C₁₅ alkyl group or a C₅ to C₁₀ aryl group, for example a radical chosen from among the methylene-4-4-bicyclohexyl radical, the radical derived from isophorone diisocyanate, the 2,4- and 2,6-tolylenes, 1,5-naphthylene, p-phenylene and 4,4N-biphenylenemethane, or Y represents a linear or branched C₁ to C₄₀ alkylene radical, or a C₄ to C₁₂ cycloalkylene radical, or
Y represents a polyurethane or polyurea sequence corresponding to the condensation of several diisocyanate molecules with one or more molecules of coupling agents of the diol or diamine type, corresponding to the formula: 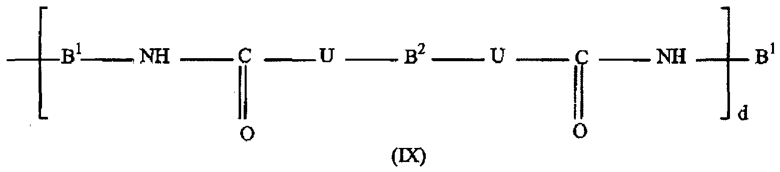 in which B¹ is a group chosen from among the groups given hereinabove for Y, U is -O- or -NH-, and B² is chosen from among:
• linear or branched C₁ to C₄₀ alkylene groups, which possibly can bear an ionizable group such as a carboxylic or sulfonic acid group, or a neutralizable or quaternizable tertiary amine group,
• C₅ to C₁₂ cycloalkylene groups, possibly bearing alkyl substituents, for example one to three methyl or ethyl groups, or alkylene, for example the diol radical: cyclohexanedimethanol,
• phenylene groups which possibly can bear C₁ to C₃ alkyl substituents, and
• groups of formula: 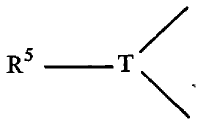
in which T is a trivalent hydrocarbon radical which can contain one or more hetero atoms such as oxygen, sulfur and nitrogen and R⁵ is a polyorganosiloxane chain or linear or branched C₁ to C₅₀ alkyl chain.

18. Emulsion according to anyone of Claims 11 to 17, **characterized in that** the gelling polymer furthermore comprises a hydrocarbon moiety comprising two groups capable of establishing hydrogen interactions, chosen from among the ester, amide, sulfonamide, carbamate, thiocarbamate, urea, thiourea, oxamido, guanidino and biguanidino groups, or combinations thereof.

19. Emulsion according to claim 18, **characterized in that** the copolymer is a block copolymer, a sequenced copolymer or a grafted copolymer.

20. Emulsion according to anyone of the preceding claims, **characterized in that** the polymer represents from 0.5 to 80% of the total weight of the emulsion, preferably from 2 to 60% and better from 5 to 40% of the total weight of the emulsion.

21. Emulsion according to claim 1, **characterized in that** the liquid fatty phase also contains a non-silicone oil.

22. Emulsion according to anyone of the preceding claims, **characterized in that** the gelling polymer/silicone oil(s) mass ratio is from 0.1 to 50%.

23. Emulsion according to anyone of the preceding claims, **characterized in that** the liquid fatty phase represents from 5 to 98.4% of the total weight of the emulsion and better from 20 to 75% of the total weight of the emulsion.

24. Emulsion according to anyone of the preceding claims, **characterized in that** it exists in the form of a water-in-oil (W/O), oil-in-water (O/W) emulsion, or a multiple emulsion, such as a triple oil-in-water-in-oil or water-in-oil-in-water emulsion.

25. Emulsion according to anyone of the preceding claims, **characterized in that** the aqueous phase represents from 1 to 94.4% of the total weight of the emulsion, preferably from 5 to 80%, and better from 20 to 60% of the total weight of the emulsion.

26. Emulsion according to anyone of the preceding claims, **characterized in that** it comprises, in addition, at least one cosmetic or dermatological active substance.

27. Emulsion according to claim 26, **characterized in that** the active substance is chosen from among essential oils, vitamins, moisturizers, filters, healing agents and ceramides.

28. Emulsion according to anyone of the preceding claims, **characterized in that** it comprises at least one additive chosen from among gelling agents of the aqueous phase, antioxidants, preservatives, fragrances, electrolytes, polymers which are liposoluble, hydrosoluble or dispersible in
the medium, in particular hydrocarbons such as polyalkylenes or vinyl polylaurate, gelling agents of the liquid fatty phase, neutralizers, gums, resins, surfactants such as trioleyl phosphate, additional cosmetic or dermatological active substances chosen, for example, from within the group consisting of emollients, moisturizers such as glycerin and sodium hyaluronate, vitamins such as vitamins A, C, D, E and F, essential fatty acids, sunscreens, dispersants, lipid vesicles and mixtures thereof.

29. Emulsion according to anyone of the preceding claims, **characterized in that** it furthermore comprises an amphiphilic compound liquid at room temperature, with a hydrophilic/lipophilic balance value less than 12.

30. Emulsion according to anyone of the preceding claims, in which the coloring agent is present in the ratio of from 0.01 to 50% of the total weight of the emulsion, preferably from 5 to 30%.

31. Emulsion according to any one of the preceding claims, **characterized in that** it exists in the form of a rigid gel.

32. Emulsion according to anyone of the preceding claims, **characterized in that** it exists in cast form.

33. Emulsion according to anyone of the preceding claims, **characterized in that** it exists in the form of cake mascara, eyeliner, foundation, lipstick, blusher, makeup-removal product, body-makeup product, eye shadow or face powder, concealer product, sun protection, product for care of the face or the body.

34. Cosmetic process for care, and/or makeup and/or treatment of the keratinous matter in humans, **characterized in that** it comprises the application on the keratinous matter of a cosmetic emulsion as defined in anyone of the preceding claims.

35. Cosmetic process for improving the properties of non-transfer and/or suppleness and/or flexibility and/or staying power of a deposit, **characterized in that** said deposit is obtained from a cosmetic emulsion as defined in anyone of claims 1 to 33.

## Patentansprüche

1. Feste kosmetische Emulsion zur Pflege und/oder als Make-up, **dadurch gekennzeichnet, dass** (i) sie bei Raumtemperatur und atmosphärischem Druck nicht unter ihrem eigenen Gewicht fließt, (ii) sie eine wässrige Phase und eine flüssige Fettphase umfasst, wobei eine in der anderen dispergiert ist, wobei die flüssige Fettphase mindestens ein flüchtiges Silikonöl umfasst, das von 20 bis 50 Gewichts-% des Gesamtgewichts der Emulsion ausmacht und aus der Gruppe ausgewählt ist, bestehend aus den folgenden Verbindungen: Octyltrimethicon, Hexyltrimethicon, Decamethylcyclopentasiloxan D5, Octamethylcyclotetrasiloxan D4, Dodecamethylcyclohexasiloxan D6, Heptamethyloctyltrisiloxan, Decamethyltetrasiloxan, Dodecamethylpentasiloxan, 1,5 cSt Polydimethylsiloxan, 2 cSt Polydimethylsi-loxan, 3 cSt Polymethylsiloxan, 5 cSt Polydimethylsiloxan, wobei cSt eine Visko-sitätseinheit ist, die äquivalent zu 10⁻⁶m².s⁻¹ ist, und deren Mischungen, und (iii) die flüssige Fettphase strukturiert ist mit mindestens einem gelierenden Polymer (Homopolymer oder Copolymer) mit einem Gewichtsmittel des Molekulargewichts von 500 bis 500.000, das mindestens eine Struktureinheit enthält, umfassend:
- mindestens eine Polyorganosiloxangruppe, die aus 1 bis 1000 Organosiloxaneinheiten in der Kette der Struktureinheit oder in der Form einer Pfropfung besteht, und
- mindestens zwei Gruppen, die in der Lage sind, Wasserstoffwechselwirkungen zu etablieren, ausgewählt aus Ester, Amid-, Sulfonamid-, Carbamat-, Thiocarbamat-, Harnstoff-, Thioharnstoff-, Oxamido-, Guanidino-, Biguanidinogruppen und deren Kombinationen, unter der Bedingung, dass mindestens eine der Gruppen von einer Estergruppe verschieden ist,
wobei das Polymer bei Raumtemperatur fest ist und bei einer Temperatur von 25 bis 250 °C in der flüssigen Fettphase löslich ist, die wässrige Phase, die flüssige Fettphase und das gelierende Polymer ein physiologisch akzeptables Medium bilden, und **dadurch gekennzeichnet, dass** sie ein Färbemittel umfasst.

2. Emulsion nach Anspruch 1, **dadurch gekennzeichnet, dass** die Fettphase lediglich aus einem oder mehreren flüchtigen Silikonölen besteht mit vorzugsweise einem Flammpunkt von gleich oder größer als 40 °C und/oder einer Viskosität von weniger als 8 cSt, wobei cSt eine Viskositätseinheit ist, die äquivalent zu 10⁻⁶m².s⁻¹ ist.

3. Emulsion nach Anspruch 1 oder 2, **dadurch gekennzeichnet, dass** das flüchtige Öl einen Flammpunkt von mehr als 60 °C aufweist.

4. Emulsion nach einem der Ansprüche 1 bis 3, **dadurch gekennzeichnet, dass** das flüchtige Öl einen Flammpunkt von kleiner oder gleich 135 °C aufweist.

5. Emulsion nach einem der Ansprüche 1 bis 4, **dadurch gekennzeichnet, dass** sie zusätzlich feste Teilchen umfasst, ausgewählt aus Füllstoffen, Pigmenten, perlmuttartigen oder anderen Teilchen und deren Mischungen.

6. Emulsion nach Anspruch 5, **dadurch gekennzeichnet, dass** die festen Teilchen hydrophobe Teilchen sind.

7. Emulsion nach Anspruch 6, **dadurch gekennzeichnet, dass** die festen Teilchen hydrophile Teilchen sind, die mit einem Film aus einer hydrophoben Verbindung umhüllt sind.

8. Emulsion nach Anspruch 5, **dadurch gekennzeichnet, dass** die festen Teilchen hydrophile Teilchen sind und die Emulsion ferner mindestens ein amphiphiles Silikon umfasst.

9. Emulsion nach Anspruch 6, **dadurch gekennzeichnet, dass** die festen Teilchen aus Pulvern oder Fasern von hydrophoben Polymeren oder Copolymeren bestehen.

10. Emulsion nach Anspruch 5 bis 9, **dadurch gekennzeichnet, dass** die Teilchen anorganische Pigmente sind, ausgewählt aus Zinkoxiden, Eisenoxiden, Titanoxiden und deren Mischungen.

11. Emulsion nach einem der vorhergehenden Ansprüche, **dadurch gekennzeichnet, dass** das Polymer mindestens eine Struktureinheit der Formel (III) oder (IV) umfasst: 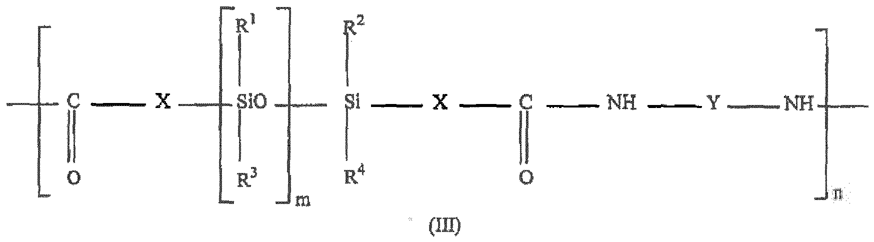 oder 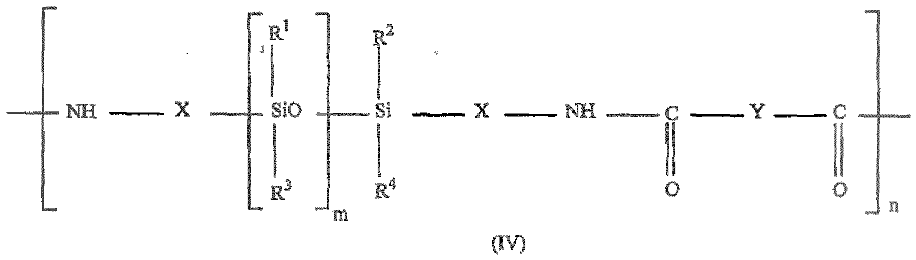 in denen
1) R¹, R², R³ und R⁴ identisch oder verschieden sind und eine Gruppe darstellen, ausgewählt aus:
- linearen, verzweigten oder cyclischen, gesättigten oder ungesättigten C₁- bis C₄₀-Kohlenwasserstoffgruppen, die in ihrer Kette ein oder mehrere Sauer- stoff-, Schwefel- und/oder Stickstoffatome enthalten können und die teilweise oder vollständig mit Fluoratomen substituiert sein können,
- C₆- bis C₁₀-Arylgruppen, die eventuell mit einer oder mehreren C₁- bis C₄-Alkylgruppen substituiert sind,
- Polyorganosiloxanketten, die ein oder mehrere Sauerstoff-, Schwefel- und/oder Stickstoffatome enthalten können oder nicht;
2) die Gruppen X identisch oder verschieden sind und eine lineare oder verzweigte C₁- bis C₃₀-Alkylendiylgruppe darstellen, die in ihrer Kette ein oder mehrere Sauerstoff- und/oder Stickstoffatome enthalten kann;
3) Y eine gesättigte oder ungesättigte, C₁ bis C₅₀ lineare oder verzweigte divalente Alkylen-, Arylen-, Cycloalkylen-, Alkylarylen- oder Arylalkylengruppe ist, die ein oder mehrere Sauerstoff-, Schwefel- und/oder Stickstoffatome umfassen kann und/oder als Substituent eines der folgenden Atome oder Gruppen von Atomen trägt: Fluor, Hydroxy, C₃- bis C₈-Cycloalkyl, C₁- bis C₄₀-Alkyl, C₅- bis C₁₀-Aryl, Phenyl eventuell substituiert mit 1 bis 3 C₁- bis C₃-Alkylgruppen, C₁- bis C₃-Hydroxyalkyl und C₁- bis C₆-Aminoalkyl, oder
4) Y eine Gruppe darstellt, die der folgenden Formel entspricht: 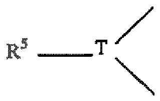 in welcher
- T eine lineare oder verzweigte, gesättigte oder ungesättigte, trivalente oder tetravalente C₃ bis C₂₄ Kohlenwasserstoffgruppe darstellt, die eventuell substituiert ist mit einer Polyorganosiloxankette, die ein oder mehrere Atome enthalten kann, ausgewählt aus O, N und S, oder T ein trivalentes Atom darstellt, ausgewählt aus N, P und Al, und
- R⁵ eine lineare oder verzweigte C₁- bis C₅₀-Alkylgruppe oder eine Polyorganosiloxankette darstellt, die eine oder mehrere Ester-, Amid-, Urethan-, Thiocarbamat-, Harnstoff-, Thioharnstoff- und/oder Sulfonamidgruppen umfassen kann, die mit einer anderen Kette des Polymers verbunden oder nicht verbunden sein können,
5) n eine ganze Zahl von 2 bis 500, vorzugsweise von 2 bis 200 ist und m eine ganze Zahl von 1 bis 1000, vorzugsweise von 1 bis 700 und noch weiter bevorzugt von 6 bis 200 ist.

12. Emulsion nach Anspruch 11, **dadurch gekennzeichnet, dass** X und/oder Y eine Alkylengruppe darstellen, die in ihrem Alkylenabschnitt mindestens eines der folgenden Elemente enthält:
1) 1 bis 5 Amid-, Harnstoff- oder Carbamatgruppen,
2) eine C₅- oder C₆-Cycloalkylgruppe und
3) eine Phenylengruppe, die eventuell substituiert ist mit 1 bis 3 identischen oder verschiedenen C₁- bis C₃-Alkylgruppen und/oder substituiert ist mit mindestens einem Element, ausgewählt aus der Gruppe bestehend aus:
- einer Hydroxygruppe,
- einer C₃- bis C₈-Cycloalkylgruppe,
- einer bis drei C₁- bis C₄₀-Alkylgruppen,
- einer Phenylgruppe, die eventuell substituiert ist mit einer bis drei C₁- bis C₃-Alkylgruppen,
- einer C₁- bis C₃-Hydroxyalkylgruppe und
- einer C₁- bis C₆-Aminoalkylgruppe.

13. Emulsion nach Anspruch 11, **dadurch gekennzeichnet, dass** Y darstellt: 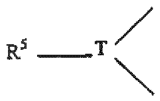 wobei R⁵ eine Polyorganosiloxankette darstellt und T eine Gruppe der folgenden Formel darstellt: 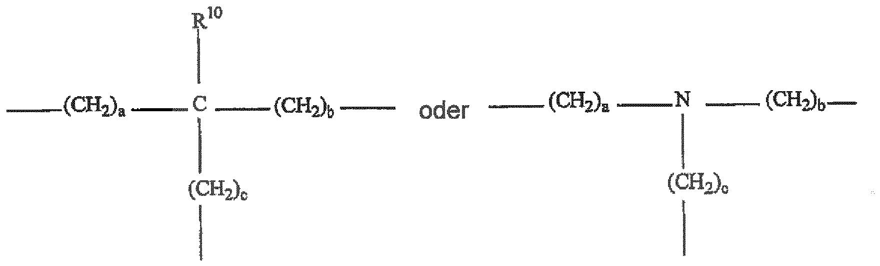 in welcher a, b und c unabhängig ganze Zahlen von 1 bis 10 sind und R¹⁰ ein Wasserstoffatom oder eine Gruppe ist wie die als R¹, R², R³ und R⁴ in Anspruch 17 definierten.

14. Emulsion nach Anspruch 11, **dadurch gekennzeichnet, dass** R¹, R², R³ und R⁴ unabhängig eine lineare oder verzweigte C₁- bis C₄₀-Alkylgruppe, vorzugsweise eine CH₃-, C₂H₅-, n-C₃H₇- oder Isopropylgruppe, eine Polyorganosiloxankette oder eine Phenylgruppe, die eventuell mit einer bis drei Methyl- oder Ethylgruppen substituiert ist, darstellen.

15. Emulsion nach einem der Ansprüche 1 bis 10, **dadurch gekennzeichnet, dass** das gelierende Polymer mindestens eine Struktureinheit der folgenden Formel umfasst: 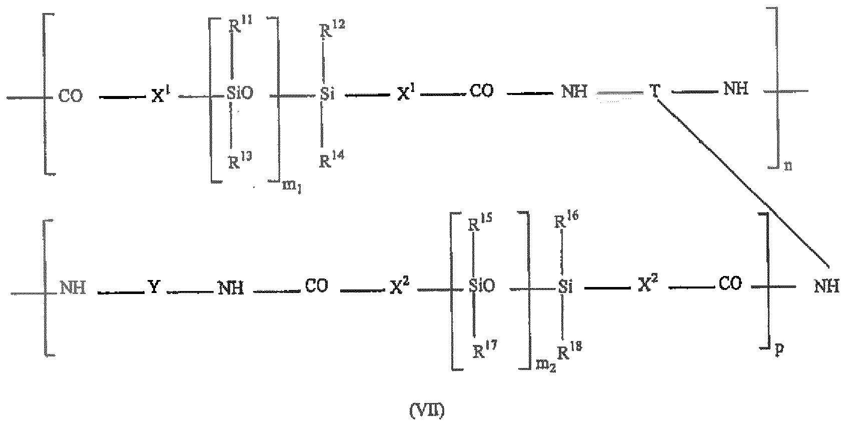 in welcher X¹ und X², die identisch oder verschieden sind, die für X in Anspruch 11 angegebene Bedeutung aufweisen, n, Y und T wie in Anspruch 11 definiert sind, R¹¹ bis R¹⁸ Gruppen sind, die aus derselben Gruppe ausgewählt sind wie R¹ bis R⁴ in Anspruch 11, m₁ und m₂ Zahlen sind, die in den Bereich von 1 bis 1000 fallen, und p eine ganze Zahl von 2 bis 500 ist.

16. Emulsion nach Anspruch 15, **dadurch gekennzeichnet, dass**:
- p im Bereich von 1 bis 25, vorzugsweise von 1 bis 7 liegt,
- R¹¹ bis R¹⁸ Methylgruppen sind,
- T einer der folgenden Formeln entspricht: 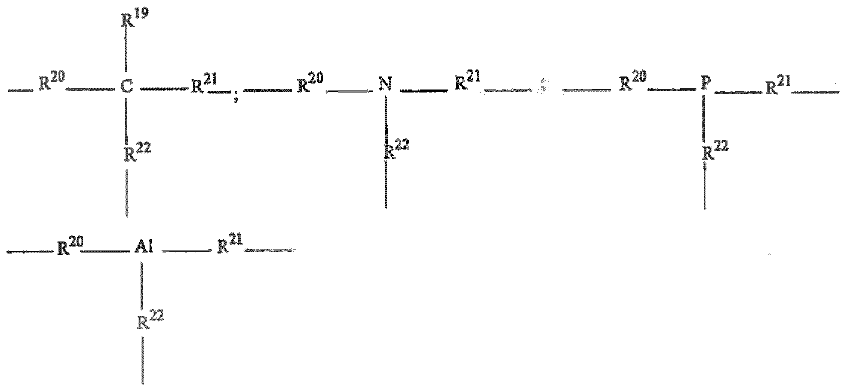 in welchen R¹⁹ ein Wasserstoffatom oder eine Gruppe ist, ausgewählt aus den Gruppen, wie sie für R¹ bis R⁴ definiert sind, und R²⁰, R²¹ und R²² unabhängig lineare oder verzweigte Alkylengruppen sind, und weiter bevorzugt der Formel: 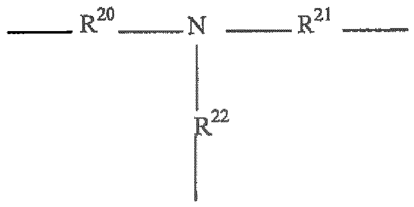 wobei R²⁰, R²¹ und R²² insbesondere -CH₂-CH₂- darstellen,
- m₁ und m₂ im Bereich von 15 bis 500 und weiter bevorzugt von 15 bis 45 liegen,
- X¹ und X² für -(CH₂)₁₀-stehen und
- T für -CH₂- steht.

17. Emulsion nach einem der Ansprüche 1 bis 10, **dadurch gekennzeichnet, dass** das gelierende Polymer mindestens eine Struktureinheit umfasst, die der folgenden Formel entspricht: 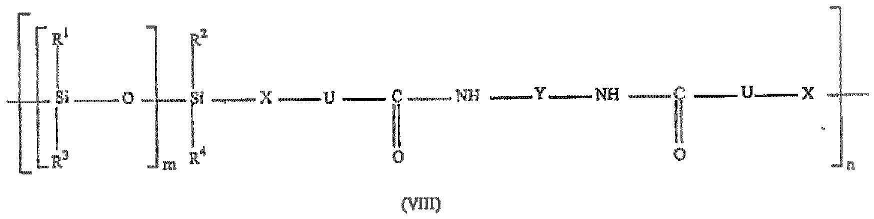 in welcher R¹, R², R³, R⁴, X, Y, m und n die oben für Formel (I) in Anspruch 11 angegebenen Bedeutungen aufweisen und U für -O- oder -NH- steht oder
Y eine cycloaliphatische oder aromatische C₅- bis C₁₂-Gruppe darstellt, die substituiert sein kann mit einer C₁- bis C₁₅-Alkylgruppe oder einer C₅- bis C₁₀-Arylgruppe, beispielsweise ein Radikal ausgewählt aus dem Methylen-4-4-bicyclo-hexylradikal, dem Radikal das abgeleitet ist von Isophorondiisocyanat, den 2,4- und 2,6-Tolylenen, 1,5-Naphthylen, p-Phenylen und 4,4N-Biphenylenmethan, oder Y ein lineares oder verzweigtes C₁- bis C₄₀-Alkylenradikal oder ein C₄- bis C₁₂-Cycloalkylenradikal darstellt, oder
Y eine Polyurethan- oder Polyharnstoffsequenz darstellt entsprechend der Kondensation von mehreren Diisocyanatmolekülen mit einem oder mehreren Molekülen von Kopplungsreagenzien vom Diol- oder Diamintyp, entsprechend der Formel: 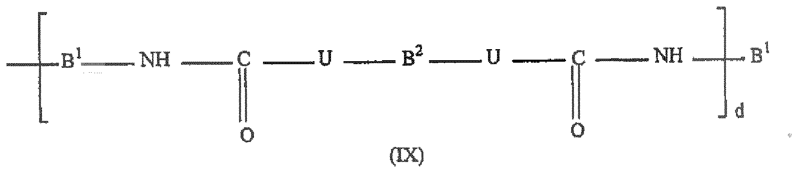 in welcher B¹ eine Gruppe ist, ausgewählt aus den oben für Y angegebenen Gruppen, U gleich -O- oder -NH- ist und B² ausgewählt ist aus:
• linearen oder verzweigten C₁- bis C₄₀-Alkylengruppen, die eventuell eine ionisierbare Gruppe wie eine Carboxyl- oder Sulfonsäuregruppe oder eine neutralisierbare oder quaternisierbare tertiäre Amingruppe tragen können,
• C₅- bis C₁₂-Cycloalkylengruppen, die eventuell Alkylsubstiuenten, beispielsweise eine bis drei Methyl- oder Ethylgruppen, oder Alkylen, zum Beispiel das Diolradikal: Cyclohexandimethanol, tragen,
• Phenylengruppen, die eventuell C₁- bis C₃-Alkylsubstituenten tragen können,
und
• Gruppen der Formel 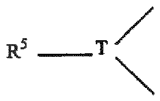
in welcher T ein trivalentes Kohlenwasserstoffradikal ist, der ein oder mehrere Heteroatome wie Sauerstoff, Schwefel und Stickstoff enthalten kann, und R⁵ eine Polyorganosiloxankette oder eine lineare oder verzweigte C₁- bis C₅₀-Alkylkette ist.

18. Emulsion nach einem der Ansprüche 11 bis 17, **dadurch gekennzeichnet, dass** das gelierende Polymer ferner eine Kohlenwasserstoffstruktureinheit umfasst, umfassend zwei Gruppen, die in der Lage sind, Wasserstoffwechselwirkungen zu etablieren, ausgewählt aus Ester-, Amid-, Sulfonamid-, Carbamat-, Thiocarbamat-, Harnstoff-, Thioharnstoff-, Oxamido-, Guanidino- und Biguanidinogruppen oder deren Kombinationen.

19. Emulsion nach Anspruch 18, **dadurch gekennzeichnet, dass** das Copolymer ein Blockcopolymer, ein Sequenzcopolymer oder ein Pfropfcopolymer ist.

20. Emulsion nach einem der vorhergehenden Ansprüche, **dadurch gekennzeichnet, dass** das Polymer von 0,5 bis 80 % des Gesamtgewichts der Emulsion, vorzugsweise von 2 bis 60 % und weiter bevorzugt von 5 bis 40 % des Gesamtgewichts der Emulsion ausmacht.

21. Emulsion nach Anspruch 1, **dadurch gekennzeichnet, dass** die flüssige Fettphase auch ein Nichtsilikonöl enthält.

22. Emulsion nach einem der vorhergehenden Ansprüche, **dadurch gekennzeichnet, dass** das Massenverhältnis gelierendes Polymer / Silikonöl(e) von 0,1 bis 50 % beträgt.

23. Emulsion nach einem der vorhergehenden Ansprüche, **dadurch gekennzeichnet, dass** die flüssige Fettphase von 5 bis 98,4 % des Gesamtgewichts der Emulsion und weiter bevorzugt von 20 bis 75 % des Gesamtgewichts der Emulsion ausmacht.

24. Emulsion nach einem der vorhergehenden Ansprüche, **dadurch gekennzeichnet, dass** sie in der Form einer Wasser-in-Öl-Emulsion (W/O- Emulsion), Öl-in-Wasser-Emulsion (O/W-Emulsion) oder einer multiplen Emulsion wie einer Öl-in-Wasser-in-Öl- oder Wasser-in-Öl-in-Wasser-Dreifachemulsion existiert.

25. Emulsion nach einem der vorhergehenden Ansprüche, **dadurch gekennzeichnet, dass** die wässrige Phase von 1 bis 94,4 % des Gesamtgewichts der Emulsion, vorzugsweise von 5 bis 80 % und weiter bevorzugt von 20 bis 60 % des Gesamtgewichts der Emulsion ausmacht.

26. Emulsion nach einem der vorhergehenden Ansprüche, **dadurch gekennzeichnet, dass** sie zusätzlich mindestens eine kosmetische oder dermatologisch aktive Substanz umfasst.

27. Emulsion nach Anspruch 26, **dadurch gekennzeichnet, dass** die aktive Substanz ausgewählt ist aus essenziellen Ölen, Vitaminen, Feuchtigkeitszusätzen, Filtern, heilenden Substanzen und Ceramiden.

28. Emulsion nach einem der vorhergehenden Ansprüche, **dadurch gekennzeichnet, dass** sie mindestens ein Additiv, ausgewählt aus Geliermitteln der wässrigen Phase, Antioxidationsmitteln, Konservierungsstoffen, Duftstoffen, Elektrolyten, Polymeren, die lipolöslich, hydrolöslich oder in dem Medium dispergierbar sind, insbesondere Kohlenwasserstoffen wie Polyalkylenen oder Vinylpolylaurat, Geliermitteln der flüssigen Fettphase, Neutralisationsmitteln, Gummis, Harzen, Tensiden wie Trioleylphosphat, zusätzliche kosmetische oder dermatologisch aktive Substanzen, ausgewählt aus beispielsweise der Gruppe bestehend aus Weichmachern, Feuchtigkeitszusätzen wie Glycerin und Natriumhyaluronat, Vitamine wie Vitamin A, C, D, E und F, essenzielle Fettsäuren, Sonnenschutzmittel, Dispergiermittel, Lipidvesikel und deren Mischungen umfasst.

29. Emulsion nach einem der vorhergehenden Ansprüche, **dadurch gekennzeichnet, dass** sie ferner eine bei Raumtemperatur flüssige amphiphile Verbindung mit einem HLB-Wert (hydrophilic/lipophilic balance) von weniger als 12 umfasst.

30. Emulsion nach einem der vorhergehenden Ansprüche, bei der das Färbemittel in dem Anteil von 0,01 bis 50 % des Gesamtgewichts der Emulsion, vorzugsweise von 5 bis 30 % vorhanden ist.

31. Emulsion nach einem der vorhergehenden Ansprüche, **dadurch gekennzeichnet, dass** sie in der Form eines festen Gels existiert.

32. Emulsion nach einem der vorhergehenden Ansprüche, **dadurch gekennzeichnet, dass** sie als gegossenes Formstück existiert.

33. Emulsion nach einem der vorhergehenden Ansprüche, **dadurch gekennzeichnet, dass** sie in der Form von Cake-Maskara, Eyeliner, Foundation, Lippenstift, Rouge, Make-up-Entfemungsprodukt, Körper-Make-up-Produkt, Lidschatten oder Gesichtspuder, Concealer-Produkt, Sonnenschutz, Pflegeprodukt für das Gesicht oder den Körper existiert.

34. Kosmetisches Verfahren zur Pflege und/oder Make-up und/oder Behandlung von Keratinmaterial beim Menschen, **dadurch gekennzeichnet, dass** es die Anwendung einer kosmetischen Emulsion, wie sie in einem der vorhergehenden Ansprüche definiert ist, auf das Keratinmaterial umfasst.

35. Kosmetisches Verfahren zur Verbesserung des Nichtübertragens und/oder der Geschmeidigkeit und/oder der Flexibilität und/oder der Haltbarkeit eines Auftrags, **dadurch gekennzeichnet, dass** der Auftrag erhalten wird aus einer kosmetischen Emulsion, wie sie in einem der Ansprüche 1 bis 33 definiert ist.

## Revendications

1. Émulsion solide cosmétique de soin et/ou de maquillage, **caractérisée en ce que** (i) elle ne s'écoule pas sous son propre poids à température ambiante et à pression atmosphérique, (ii) elle comprend une phase aqueuse et une phase grasse liquide dispersées l'une dans l'autre, ladite phase grasse liquide comprenant au moins une huile de silicone volatile représentant de 20 à 50 % en poids du poids total de l'émulsion et étant choisie dans le groupe constitué des composés suivants : octyltriméthicone, hexyltriméthicone, décaméthyl-cyclopentasiloxane D5, octaméthyl-cyclotétrasiloxane D4, dodécaméthyl-cyclohexasiloxane D6, heptaméthyl-octyltrisiloxane, décaméthyl-tétrasiloxane, dodécaméthyl-pentasiloxane, polydiméthylsiloxane de 1,5 cSt, polydiméthylsiloxane de 2 cSt, polyméthylsiloxane de 3 cSt, polydiméthylsiloxane de 5 cSt, cSt étant une unité de viscosité équivalant à 10⁻⁶m²•s⁻¹, et les mélanges de ceux-ci, et (iii) ladite phase grasse liquide est structurée avec au moins un polymère (homopolymère ou copolymère) gélifiant ayant une masse moléculaire moyenne en poids située dans la plage de 500 à 500 000, contenant au moins un groupement comprenant :
- au moins un groupe polyorganosiloxane, composé de 1 à 1 000 unités organosiloxane dans la chaîne du groupement ou sous la forme d'un greffon, et
- au moins deux groupes capables de créer des interactions hydrogène, choisis parmi les groupes ester, amide, sulfonamide, carbamate, thiocarbamate, urée, thiourée, oxamido, guanidino, biguanidino, et les combinaisons de ceux-ci, à condition qu'au moins l'un des groupes soit autre qu'un groupe ester,
le polymère étant solide à température ambiante et soluble dans la phase grasse liquide à une température de 25 à 250 °C, la phase aqueuse, la phase grasse liquide et le polymère gélifiant formant un milieu physiologiquement acceptable, et **caractérisée en ce qu'**elle comprend un colorant.

2. Émulsion selon la revendication 1, **caractérisée en ce que** la phase grasse est uniquement constituée d'une huile (d'huiles) de silicone volatile(s) ayant de préférence un point d'éclair supérieur ou égal à 40 °C et/ou une viscosité inférieure à 8 cSt, cSt étant une unité de viscosité équivalant à 10⁻⁶m² • s⁻¹.

3. Émulsion selon la revendication 1 ou 2, **caractérisée en ce que** l'huile volatile a un point d'éclair dépassant 60 °C.

4. Émulsion selon l'une quelconque des revendications 1 à 3, **caractérisée en ce que** l'huile volatile a un point d'éclair inférieur ou égal à 135 °C.

5. Émulsion selon l'une quelconque des revendications 1 à 4, **caractérisée en ce qu'**elle comprend par ailleurs des particules solides choisies parmi les charges ; les pigments, les composes nacrés ou autres ; et les mélanges de ceux-ci.

6. Émulsion selon la revendication 5, **caractérisée en ce que** les particules solides sont des particules hydrophobes.

7. Émulsion selon la revendication 6, **caractérisée en ce que** les particules solides sont des particules hydrophiles, enrobées dans un film de composé hydrophobe.

8. Émulsion selon la revendication 5, **caractérisée en ce que** les particules solides sont des particules hydrophiles et l'émulsion comprend en outre au moins une silicone amphiphile.

9. Émulsion selon la revendication 6, **caractérisée en ce que** les particules solides sont constituées de poudres ou de fibres de polymères ou de copolymères hydrophobes.

10. Émulsion selon la revendication 5 à 9, **caractérisée en ce que** les particules sont des pigments inorganiques choisis parmi les oxydes de zinc, les oxydes de fer, les oxydes de titane et les mélanges de ceux-ci.

11. Émulsion selon l'une quelconque des revendications précédentes, **caractérisée en ce que** le polymère comprend au moins un groupement de formule (III) ou (IV) : 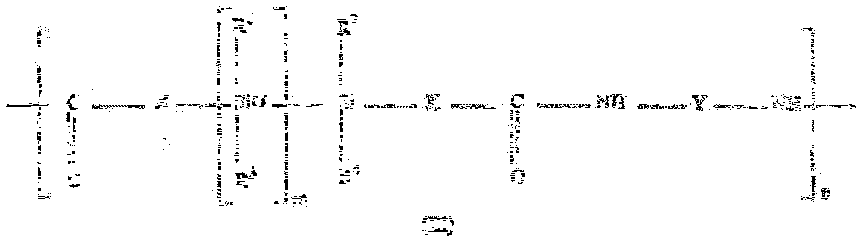 ou 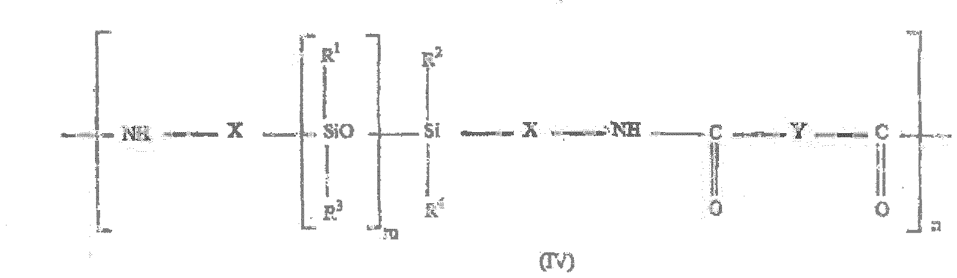 formules dans lesquelles
1) R¹, R², R³ et R⁴, identiques ou différents, représentent un groupe choisi parmi :
- des groupes hydrocarbures en C₁ à C₄₀ linéaires, ramifiés ou cycliques, saturés ou insaturés, qui peuvent contenir dans leur chaîne un ou plusieurs atomes d'oxygène, de soufre et/ou d'azote, et qui peuvent être partiellement ou totalement substitués par des atomes de fluor,
- des groupes aryles en C₆ à C₁₀, éventuellement substitués par un ou plusieurs groupes alkyles en C₁ à C₄,
- des chaînes polyorganosiloxane contenant ou ne contenant pas un ou plusieurs atomes d'oxygène, de soufre et/ou d'azote;
2) les groupes X, identiques ou différents, représentent un groupe alkylènediyle en C₁ à C₃₀, linéaire ou ramifié, qui peut contenir dans sa chaîne un ou plusieurs atomes d'oxygène et/ou d'azote ;
3) Y représente un groupe alkylène, arylène, cycloalkylène, alkylarylène ou arylalkylène divalent en C₁ à C₅₀, saturé ou insaturé, linéaire ou ramifié, qui peut comprendre un ou plusieurs atomes d'oxygène, de soufre et/ou d'azote, et/ou porter en tant que substituant l'un des atomes ou groupes d'atomes suivants : fluor, hydroxy, cycloalkyle en C₃ à C₈, alkyle en C₁ à C₄₀, aryle en C₅ à C₁₀, phényle éventuellement substitué par 1 à 3 groupes alkyles en C₁ à C₃, hydroxyalkyles en C₁ à C₃ et aminoalkyles en C₁ à C₆, ou
4) Y représente un groupe correspondant à la formule : 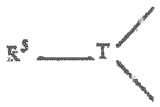 dans laquelle
- T représente un groupe hydrocarbure trivalent ou tétravalent, linéaire ou ramifié, saturé ou insaturé, en C₃ à C₂₄, éventuellement substitué par une chaîne polyorganosiloxane, qui peut contenir un ou plusieurs atomes choisis parmi 0, N et S, ou T représente un atome trivalent choisi parmi N, P et Al, et
- R⁵ représente un groupe alkyle en C₁ à C₅₀, linéaire ou ramifié, ou une chaîne polyorganosiloxane, qui peut comprendre un ou plusieurs groupes ester, amide, uréthane, thiocarbamate, urée, thiourée et/ou sulfonamide qui peut être reliée ou non reliée à une autre chaîne du polymère, 5) n représente un nombre entier situé dans la plage de 2 à 500, de préférence de 2 à 200, et m représente un nombre entier situé dans la plage de 1 à 1 000, de préférence de 1 à 700, et encore plus préférablement de 6 à 200.

12. Émulsion selon la revendication 11, **caractérisée en ce que** X et/ou Y représentent un groupe alkylène contenant dans sa partie alkylène au moins l'un des éléments suivants :
1) 1 à 5 groupes amide, urée ou carbamate.
2) un groupe cycloalkyle en C₅ ou C₆, et
3) un groupe phénylène éventuellement substitué par 1 à 3 groupes alkyles en C₁ à C₃ identiques ou différents, et/ou substitué par au moins un élément choisi dans le groupe constitué de :
- un groupe hydroxy,
- un groupe cycloalkyle en C₃ à C₈,
- un à trois groupes alkyles en C₁ à C₄₀,
- un groupe phényle éventuellement substitué par un à trois groupes alkyles en C₁ à C₃,
- un groupe hydroxyalkyle en C₁ à C₃, et
- un groupe aminoalkyle en C₁ à C₆.

13. Émulsion selon la revendication 11, **caractérisée en ce que** Y représente : 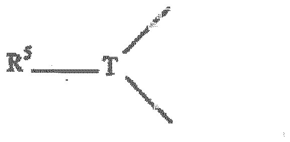 formule dans laquelle R⁵ représente une chaîne polyorganosiloxane, et T représente un groupe de formule : 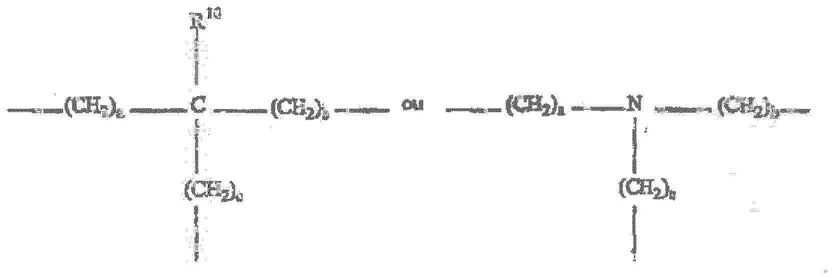 dans laquelle a, b et c représentent indépendamment des nombres entiers situés dans la plage de 1 à 10, et R¹⁰ représente un atome d'hydrogène ou un groupe tel que ceux définis pour R¹, R², R³ et R⁴, dans la revendication 17.

14. Émulsion selon la revendication 11, **caractérisée en ce que** R¹, R², R³ et R⁴ représentent indépendamment un groupe alkyle en C₁ à C₄₀, linéaire ou ramifié, de préférence un groupe CH₃, C₂H₅, n-C₃H₇ ou isopropyle, une chaîne polyorganosiloxane ou un groupe phényle éventuellement substitué par un à trois groupes méthyles ou éthyles.

15. Émulsion selon l'une quelconque des revendications 1 à 10, **caractérisée en ce que** le polymère gélifiant comprend au moins un groupement de formule : 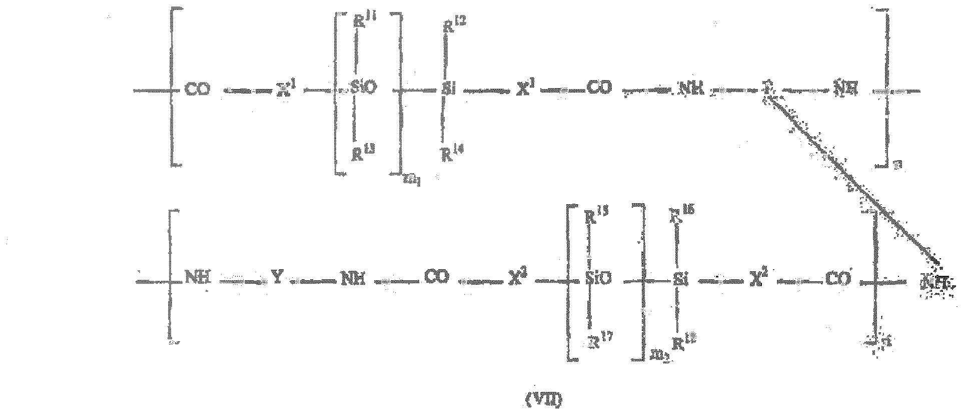 dans laquelle X¹ et X², qui sont identiques ou différents, ont la signification donnée pour X dans la revendication 11, n, Y et T sont tels que définis dans la revendication 11, R¹¹ à R¹⁸ sont des groupes choisis parmi les mêmes groupes que les groupes R¹ à R⁴ de la revendication 11, m₁ et m₂ représentent des nombres situés dans la plage de 1 à 1 000, et p représente un nombre entier situé dans la plage de 2 à 500.

16. Émulsion selon la revendication 15, **caractérisée en ce que** :
- p se situe dans la plage de 1 à 25, encore plus préférablement de 1 à 7,
- les groupes R¹¹ à R¹⁸ sont des groupes méthyles,
- T correspond à l'une des formules suivantes : 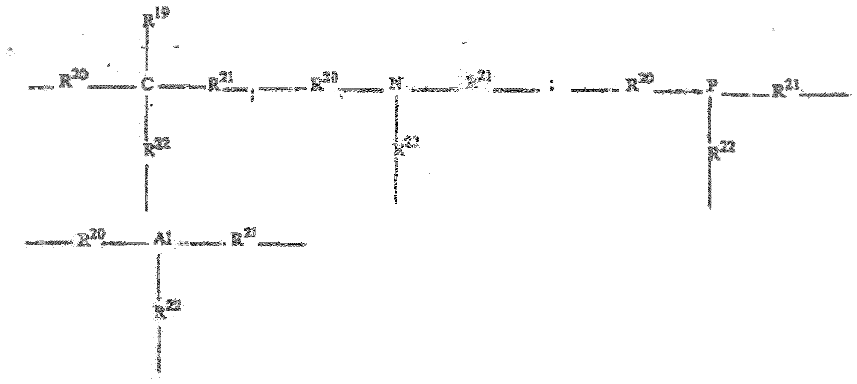 formules dans lesquelles R¹⁹ représente un atome d'hydrogène ou un groupe choisi parmi les groupes définis pour R¹ à R⁴, et R²⁰, R²¹ et R²² représentent indépendamment des groupes alkylène linéaires ou ramifiés, et répondent plus préférablement à la formule : 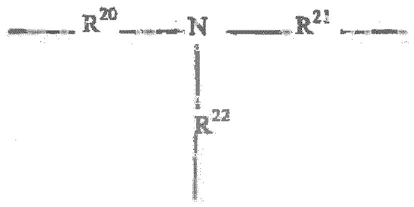 en particulier avec R²⁰, R²¹ et R²² représentant un groupe -CH₂-CH₂-,
- m₁ et m₂ sont situés dans la plage de 15 à 500, et encore plus préférablement de 15 à 45,
- X¹ et X² représentent un groupe - (CH₂)₁₀-, et
T représente un groupe -CH₂-.

17. Émulsion selon l'une quelconque des revendications 1 à 10, **caractérisée en ce que** le polymère gélifiant comprend au moins un groupement correspondant à la formule suivante : 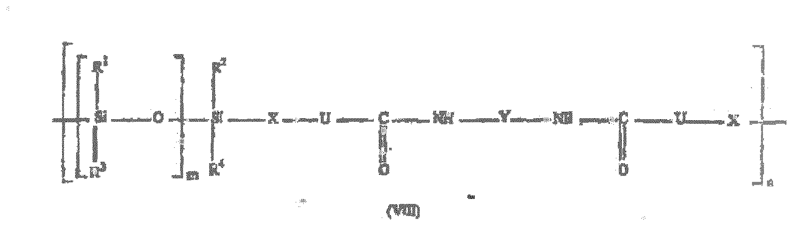 dans laquelle R¹, R², R³, R⁴, X, Y, m et n ont les significations données ci-dessus pour la formule (I) de la revendication 11, et U représente -O- ou -NH- ou
Y représente un groupe cycloaliphatique ou aromatique en C₅ à C₁₂ qui peut être substitué par un groupe alkyle en C₁ à C₁₅ ou un groupe aryle en C₅ à C₁₀, par exemple un radical choisi parmi le radical méthylène-4-4-bicyclohexyle, le radical dérivé du diisocyanate d'isophorone, les 2,4- et 2,6-tolylènes, le 1,5-naphtylène, le p-phénylène et le 4,4N-biphénylèneméthane, ou Y représente un radical alkylène en C₁ à C₄₀, linéaire ou ramifié, ou un radical cycloalkylène en C₄ à C₁₂, ou
Y représente une séquence polyuréthane ou polyurée correspondant à la condensation de plusieurs molécules de diisocyanate avec une ou plusieurs molécules d'agents de couplage de type diol ou diamine, correspondant à la formule : 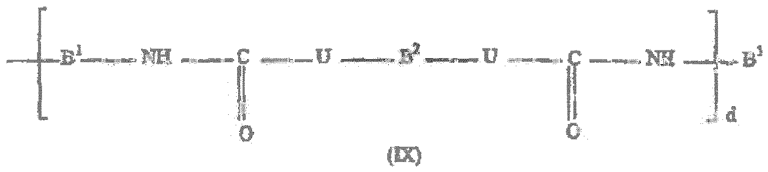 dans laquelle B¹ est un groupe choisi parmi les groupes indiqués ci-dessus pour Y, U représente -O- ou - NH-, et B² est choisi parmi :
• les groupes alkylène en C₁ à C₄₀, linéaires ou ramifiés, qui peuvent éventuellement porter un groupe ionisable tel qu'un groupe acide carboxylique ou sulfonique, ou un groupe amine tertiaire neutralisable ou quaternisable,
• les groupes cycloalkylène en C₅ à C₁₂, portant éventuellement des substituants alkyles, par exemple un à trois groupes méthyles ou éthyles, ou alkylène, par exemple le radical diol : cyclohexanediméthanol,
• les groupes phénylène qui peuvent éventuellement porter des substituants alkyles en C₁ à C₃, et
• les groupes de formule : 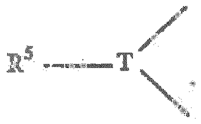
formule dans laquelle T représente un radical hydrocarbure trivalent qui peut contenir un ou plusieurs hétéroatomes tels qu'un atome d'oxygène, de soufre et d'azote et R⁵ représente une chaîne polyorganosiloxane ou une chaîne alkyle en C₁ à C₅₀, linéaire ou ramifiée.

18. Émulsion selon l'une quelconque des revendications 11 à 17, **caractérisée en ce que** le polymère gélifiant comprend en outre un groupement hydrocarbure comprenant deux groupes capables de créer des interactions hydrogène, choisis parmi les groupes ester, amide, sulfonamide, carbamate, thiocarbamate, urée, thiourée, oxamido, guanidino et biguanidino, ou leurs combinaisons.

19. Émulsion selon la revendication 18, **caractérisée en ce que** le copolymère est un copolymère en bloc, un copolymère séquencé ou un copolymère greffé.

20. Émulsion selon l'une quelconque des revendications précédentes, **caractérisée en ce que** le polymère représente de 0,5 à 80 % du poids total de l'émulsion, de préférence de 2 à 60 % et plus préférablement de 5 à 40 % du poids total de l'émulsion.

21. Émulsion selon la revendication 1, **caractérisée en ce que** la phase grasse liquide contient également une huile sans silicone.

22. Émulsion selon l'une quelconque des revendications précédentes, **caractérisée en ce que** le rapport massique polymère gélifiant/huile(s) de silicone est de 0,1 à 50 %.

23. Émulsion selon l'une quelconque des revendications précédentes, **caractérisée en ce que** la phase grasse liquide représente de 5 à 98,4 % du poids total de l'émulsion et plus préférablement de 20 à 75 % du poids total de l'émulsion.

24. Émulsion selon l'une quelconque des revendications précédentes, **caractérisée en ce qu'**elle existe sous la forme d'une émulsion d'eau dans l'huile (E/H), d'huile dans l'eau (H/E), ou d'une émulsion multiple, telle qu'une émulsion triple d'huile dans l'eau dans l'huile ou d'eau dans l'huile dans l'eau.

25. Émulsion selon l'une quelconque des revendications précédentes, **caractérisée en ce que** la phase aqueuse représente de 1 à 94,4 % du poids total de l'émulsion, de préférence de 5 à 80 %, et plus préférablement de 20 à 60 % du poids total de l'émulsion.

26. Émulsion selon l'une quelconque des revendications précédentes, **caractérisée en ce qu'**elle comprend, par ailleurs, au moins une substance active cosmétique ou dermatologique.

27. Émulsion selon la revendication 26, **caractérisée en ce que** la substance active est choisie parmi les huiles essentielles, les vitamines, les hydratants, les filtres, les cicatrisants et les céramides.

28. Émulsion selon l'une quelconque des revendications précédentes, **caractérisée en ce qu'**elle comprend au moins un additif choisi parmi les gélifiants de la phase aqueuse, les antioxydants, les conservateurs, les parfums, les électrolytes, les polymères qui sont liposolubles, hydrosolubles ou dispersibles' dans le milieu, en particulier les hydrocarbures tels que les polyalkylènes ou le polylaurate de vinyle, les gélifiants de la phase grasse liquide, les agents de neutralisation, les gommes, les résines, les tensioactifs tels que le phosphate de trioléyle, les substances actives cosmétiques ou dermatologiques additionnelles choisies, par exemple, dans le groupe constitué des émollients, les hydratants tels que la glycérine et l'hyaluronate de sodium, les vitamines telles que les vitamines A, C, D, E et F, les acides gras essentiels, les écrans solaires, les dispersants, les vésicules lipidiques et les mélanges de ceux-ci.

29. Émulsion selon l'une quelconque des revendications précédentes, **caractérisée en ce qu'**elle comprend en outre un composé amphiphile liquide à température ambiante, ayant un rapport hydro-lipophile inférieur à 12.

30. Émulsion selon l'une quelconque des revendications précédentes, dans laquelle le colorant est présent dans le rapport de 0,01 à 50 % du poids total de l'émulsion, de préférence de 5 à 30 %.

31. Émulsion selon l'une quelconque des revendications précédentes, **caractérisée en ce qu'**elle existe sous la forme d'un gel rigide.

32. Émulsion selon l'une quelconque des revendications précédentes, **caractérisée en ce qu'**elle existe sous forme moulée.

33. Émulsion selon l'une quelconque des revendications précédentes, **caractérisée en ce qu'**elle existe sous la forme d'un mascara en pain, d'un ligneur, d'un fond de teint, d'un rouge à lèvres, d'un fard à joues, d'un produit démaquillant, d'un produit de maquillage corporel, d'une ombre à paupières ou d'une poudre de visage, d'un produit correcteur, d'une protection solaire, d'un produit pour le soin du visage ou du corps.

34. Procédé cosmétique de soin et/ou de maquillage et/ou de traitement de la matière kératineuse chez les humains, **caractérisé en ce qu'**il comprend l'application sur la matière kératineuse d'une émulsion cosmétique telle que définie dans l'une quelconque des revendications précédentes.

35. Procédé cosmétique permettant d'améliorer les propriétés de non-transfert et/ou de souplesse et/ou de flexibilité et/ou de la bonne tenue d'un dépôt, **caractérisé en ce que** ledit dépôt est obtenu à partir d'une émulsion cosmétique telle que définie dans l'une quelconque des revendications 1 à 33.
